# DEMANDE DE BREVET EUROPEEN

(11) **EP 4 717 367 A1**
(43) Date de publication de la demande: **01.04.2026**
(21) Numéro de dépôt: 25204119.9
(22) Date de dépôt: 23.09.2025
(51) Int. Cl.: B08B 9/032, A61B 5/145, A61B 10/00, A61L 2/18, B08B 3/04, G01N 33/493

(54) **STATION DE NETTOYAGE POUR DISPOSITIF D'ANALYSE D'URINE**

(30) Priorité: 27.09.2024 FR 2410351
(71) Demandeur: Withings, 92130 Issy-Les-Moulineaux (FR)
(72) Inventeur: Ndaw, Inna, 92130 Issy-les-Moulineaux (FR); Menanteau, Matthieu, 92130 Issy-les-Moulineaux (FR); Dewavrin, Julius, 92130 Issy-les-Moulineaux (FR); Geffraye, Victor, 92130 Issy-les-Moulineaux (FR)
(74) Mandataire: Withings IP

(57) **Abrégé**

La présente description concerne une station de nettoyage (600) pour dispositif d'analyse d'urine, la station de nettoyage (600) comprenant :
• un récipient (610) configuré pour recevoir un liquide de nettoyage, le récipient (610) étant en outre dimensionné pour recevoir au moins en partie le dispositif d'analyse d'urine, de sorte que le dispositif d'analyse d'urine soit au contact du liquide de nettoyage,
• un chargeur (620) configuré pour alimenter en énergie le dispositif d'analyse d'urine lorsque le dispositif d'analyse d'urine est reçu au moins en partie dans le récipient (610).

## Description

La présente invention concerne une station de nettoyage pour dispositif d'analyse d'urine. L'invention concerne également un ensemble comprenant un dispositif d'analyse d'urine et une telle station de nettoyage configurée pour recevoir le dispositif d'analyse d'urine, ainsi que les méthodes de nettoyage associées. Le dispositif d'analyse d'urine comprend un boîtier configuré pour être placé entièrement dans une cuvette de toilettes.

### Etat de la technique

De nombreux paramètres biologiques sont reflétés dans l'urine d'un individu. A partir d'un échantillon d'urine, il est par exemple possible de détecter des problèmes de santé tels qu'une infection urinaire, le diabète ou une insuffisance rénale. L'échantillon d'urine peut également refléter la qualité d'une alimentation, identifier une période de fécondité ou une grossesse et détecter une prise de drogues ou de tabac. Il est alors intéressant de surveiller divers paramètres biologiques périodiquement.

Le document WO2021/175909 décrit un dispositif qui se loge sur une paroi d'une cuvette de toilettes et recueille un échantillon d'urine avant d'effectuer une analyse optique. Le dispositif comprend une station de mesure et une cartouche qui peut être retirée et remplacée de la station de mesure. La cartouche contient des réactifs réagissant avec l'urine, notamment des bandelettes de test urinaires.

Un tel dispositif d'analyse d'urine placé dans une cuvette de toilettes fait face à une problématique due au nettoyage du boitier à la suite des mictions répétées de l'utilisateur sur le boitier. En effet, la cuvette de toilettes comprend une chasse d'eau qui fait couler de l'eau de long de la cuvette. Ce flux d'eau a tendance à passer au-dessus et/ou derrière le boîtier et ne nettoie donc pas la face avant du boitier, ce qui peut présenter des problématiques de confort et d'hygiène (couleur jaunie du boitier, odeur, etc.) pour l'utilisateur.

A cet effet, le document FR2405625 (numéro de dépôt) propose un bras de fixation comprenant une rampe d'amenée d'eau configurée pour orienter un flux d'eau provenant d'une chasse d'eau de la cuvette de toilettes vers la face avant du boîtier afin de le nettoyer après chaque miction.

La problématique de nettoyage se présente également pour l'intérieur de la station de mesure dans laquelle circule de l'urine de l'utilisateur de manière répétée, ce qui peut aller jusqu'à des potentielles obstructions du fait de dépôts répétés dans le circuit fluidique de la station de mesure.

### Résumé de l'invention

La présente description se propose d'améliorer encore le nettoyage du dispositif d'analyse d'urine afin d'améliorer la durée de vie du dispositif et l'expérience des utilisateurs.

A cet effet, la présente description concerne une station de nettoyage pour dispositif d'analyse d'urine comprenant un récipient configuré pour recevoir un liquide de nettoyage, le récipient étant en outre dimensionné pour recevoir au moins en partie le dispositif d'analyse d'urine, de sorte que le dispositif d'analyse d'urine soit au contact du liquide de nettoyage ; un chargeur configuré pour alimenter en énergie le dispositif d'analyse d'urine lorsque le dispositif d'analyse d'urine est reçu au moins en partie dans le récipient.

En effet, la station de nettoyage permet de recevoir au moins en partie le dispositif d'analyse d'urine et de le tremper dans le liquide de nettoyage présent dans le récipient, ce qui permet le nettoyage d'au moins une partie de l'extérieur du dispositif, notamment autour de l'orifice de collecte.

De plus, le chargeur du dispositif de nettoyage permet d'alimenter en énergie le dispositif d'analyse d'urine, ce qui permet par exemple la recharge d'une batterie rechargeable du dispositif avant un nouveau cycle d'utilisation dans les toilettes de l'utilisateur et donc de recharger la batterie pendant une phase de nettoyage. Les tâches de recharge et de nettoyage sont effectuées en même temps, ce qui diminue le temps de non-disponibilité du dispositif d'analyse d'urine.

Cette alimentation en énergie via le chargeur peut aussi permettre d'activer une pompe arrangée dans le dispositif afin d'aspirer du liquide de nettoyage et d'ainsi nettoyer au moins en partie le circuit fluidique du dispositif. La station de nettoyage utilise ainsi la pompe du dispositif d'analyse d'urine pour nettoyer le circuit fluidique.

La station de nettoyage selon la présente description permet donc un nettoyage aisé et efficace pour l'utilisateur, ainsi qu'une alimentation électrique en parallèle du nettoyage. En particulier, le chargeur peut être un chargeur sans-fil (typiquement par induction), ce qui simplifie la mise en place du dispositif d'analyse d'urine dans la station de nettoyage (moins de geste, pas de risque d'infiltration d'eau ou de court-circuit, etc.).

Dans un mode de réalisation, le récipient reçoit de manière amovible le dispositif d'analyse d'urine.

Dans un mode de réalisation, le liquide de nettoyage est du détergent.

Dans un mode de réalisation, le récipient comprend une jauge indiquant le volume recommandé de liquide de nettoyage à verser dans le récipient.

Dans un mode de réalisation, la station est dépourvue de moyens de circulation du liquide de nettoyage dans le récipient.

Dans un mode de réalisation, le chargeur est positionné, le long de la profondeur du récipient, au moins en partie entre le fond du récipient et la jauge.

Dans un mode de réalisation, le dispositif d'analyse d'urine comprend une batterie rechargeable et le chargeur est configuré pour charger électriquement la batterie.

Dans un mode de réalisation, le chargeur est un chargeur sans fil, de sorte que la charge entre le chargeur et le dispositif d'analyse d'urine est sans fil.

Dans un mode de réalisation, le chargeur est à induction électromagnétique.

Dans un mode de réalisation, la station comprend une bobine placée en vis-à-vis d'une paroi du récipient.

Dans un mode de réalisation, la bobine est placée dans une protubérance formée dans ladite paroi.

Dans un mode de réalisation, la station comprend un port de charge connectable au dispositif d'analyse d'urine

Dans un mode de réalisation, la station comprend un câble de connexion électrique pour connecter le système de charge à un réseau électrique

Dans un mode de réalisation, la station comprend une batterie.

Dans un mode de réalisation, le récipient présente une forme cylindrique droite.

Dans un mode de réalisation, le récipient présente une forme de cône tronqué.

Dans un mode de réalisation, le récipient présente une base ovale, ronde ou carrée.

Dans un mode de réalisation, le dispositif d'analyse d'urine présente une dimension de moindre longueur selon une direction X, le récipient étant configuré pour recevoir le dispositif d'analyse d'urine de sorte que la direction X s'étende horizontalement.

Dans un mode de réalisation, la largeur du récipient selon la direction X est sensiblement égale à la dimension de moindre longueur.

Dans un mode de réalisation, le récipient est configuré pour recevoir le dispositif de sorte que l'axe X s'étende selon une direction formant un angle compris entre 20° et 70°, par exemple d'environ 45°.

Dans un mode de réalisation, le récipient comprend au moins une saillie configurée pour supporter le dispositif d'analyse d'urine.

Dans un mode de réalisation, le dispositif est uniquement soutenue par la ou les saillies dans le récipient.

Dans un mode de réalisation, l'au moins une saillie définit un volume de dégagement prévu pour recevoir l'orifice de collecte du dispositif d'analyse.

Dans un mode de réalisation, au moins une saillie est arrangée en partie basse du récipient.

Dans un mode de réalisation, le récipient comprend au moins une saillie latérale faisant saillie sensiblement horizontalement de la paroi

Dans un mode de réalisation, au moins une saillie se présente sous la forme d'un promontoire configuré pour supporter le dispositif.

Dans un mode de réalisation, la station de nettoyage comprend un couvercle configuré pour fermer au moins en partie le récipient.

Dans un mode de réalisation, le couvercle comprend au moins une cale faisant saillie du couvercle vers le récipient, chaque cale étant configurée pour maintenir le dispositif d'analyse d'urine en position dans le récipient.

Dans un mode de réalisation, le couvercle comprend un crochet configuré pour coopérer avec un moyen de fixation du dispositif d'analyse d'urine.

Dans un mode de réalisation, le couvercle comprend au moins une ouverture apte à être traversée par un bras de fixation du dispositif d'analyse d'urine.

Dans un mode de réalisation, le couvercle est formé d'un matériau au moins partiellement transparent ou translucide.

Dans un mode de réalisation, le couvercle comprend une zone de moindre épaisseur pour rendre visible un indicateur lumineux du dispositif, notamment un indicateur de charge lumineux.

La présente description concerne également un ensemble comprenant :
- un dispositif d'analyse d'urine, le dispositif d'analyse d'urine comprenant un boitier et un circuit fluidique logé à l'intérieur du boîtier, et configuré pour faire circuler de l'urine dans le dispositif d'analyse d'urine, le circuit fluidique comprenant un orifice de collecte sur le boitier,
- une station telle que définie ci-dessus,
l'ensemble étant configuré pour passer d'une configuration opérationnelle dans laquelle le dispositif d'analyse d'urine est reçu dans le récipient à une configuration séparée dans laquelle le dispositif d'analyse d'urine est à l'écart du récipient.

Dans un mode de réalisation, le dispositif est configuré pour être placé entièrement dans une cuvette de toilettes dans la configuration séparée.

Dans un mode de réalisation, la station et le dispositif sont configurés pour être placés entièrement dans la cuvette de toilettes dans la configuration opérationnelle.

Dans un mode de réalisation, la station comprend un moyen de fixation du récipient à la cuvette de toilettes.

Dans un mode de réalisation, le récipient est dimensionné pour que l'orifice de collecte soit immergé dans le liquide de nettoyage en configuration opérationnelle.

Dans un mode de réalisation, le récipient est formé par une paroi et un fond, le récipient étant dimensionné pour que l'orifice de collecte soit à l'écart d'au moins l'un parmi la paroi et le fond.

Dans un mode de réalisation, le dispositif d'analyse d'urine comprend en outre un bras de fixation à une cuvette de toilette, le bras de fixation faisant saillie au moins en partie du récipient dans la configuration opérationnelle.

Dans un mode de réalisation, le circuit fluidique comprend une pompe configurée pour aspirer du liquide dans le circuit fluidique via l'orifice de collecte.

Dans un mode de réalisation, le chargeur est configuré pour, en configuration opérationnelle, alimenter la pompe du circuit fluidique, de sorte que le liquide de nettoyage soit aspiré dans le circuit fluidique.

Dans un mode de réalisation, le chargeur est en outre configuré pour recharger une batterie du dispositif d'analyse d'urine.

Dans un mode de réalisation, le dispositif d'analyse d'urine est configuré pour passer d'une configuration d'analyse d'urine à une configuration de nettoyage, le dispositif comprenant au moins un réactif propre à réagir avec de l'urine, le circuit fluidique comprenant un orifice de drainage sur le boitier, le circuit fluidique étant configuré pour, dans la configuration d'analyse d'urine, injecter le fluide collecté par l'orifice de collecte sur le réactif, le circuit fluidique étant configuré pour, dans la configuration de nettoyage, faire circuler le fluide collecté par l'orifice de collecte directement vers l'orifice de drainage.

Dans un mode de réalisation, le dispositif d'analyse d'urine est en position de drainage pendant la configuration opérationnelle.

Dans un mode de réalisation, le circuit fluidique comprend un injecteur configuré pour injecteur de l'urine sur du réactif, dans lequel en configuration de nettoyage, le circuit fluidique comprend en série l'orifice de collecte, l'injecteur et l'orifice de purge.

Dans un mode de réalisation, le dispositif est configurée pour activer la pompe lorsque que des conditions de nettoyage prédéterminées sont remplies.

Dans un mode de réalisation, les conditions de nettoyage comprennent : la réception d'un signal envoyé par un système d'alimentation électrique du dispositif indiquant qu'il reçoit de l'énergie de la part du chargeur, et/ou la réception d'un signal envoyé par un capteur radar indiquant que la paroi du récipient est détectée, et/ou la réception d'un signal envoyé par un capteur de température indiquant qu'un changement de température sur le boitier a été détecté.

Dans un mode de réalisation, le dispositif d'analyse d'urine est configuré pour aspirer et purger le liquide de nettoyage présent dans le récipient dans la configuration opérationnelle.

Dans un mode de réalisation, le dispositif comprend un capteur radar configuré pour détecter la présente d'au moins une paroi à proximité du dispositif dans la configuration opérationnelle.

Dans un mode de réalisation, le dispositif comprend un capteur de température configuré pour détecter un changement de température au niveau du boitier dans la configuration opérationnelle.

Dans un mode de réalisation, le circuit fluidique comprend un capteur de liquide configuré pour détecter une présence de liquide dans le circuit fluidique dans la configuration opérationnelle, en réponse à l'activation de la pompe.

La présente description concerne également une méthode de nettoyage et de charge mise en oeuvre par un ensemble tel que défini ci-dessus, la méthode comprenant les étapes successives suivantes, après mise de l'ensemble de la configuration séparée à la configuration opérationnelle :
- alimentation du dispositif par le chargeur,
- nettoyage du dispositif par le liquide de nettoyage dans le récipient.

Dans un mode de réalisation, le nettoyage est le nettoyage d'au moins une partie de la paroi externe du boitier.

Dans un mode de réalisation, le nettoyage est le nettoyage du circuit fluidique.

Dans un mode de réalisation, le nettoyage comprend : l'activation de la pompe pour faire circuler du liquide de nettoyage dans le circuit fluidique.

Dans un mode de réalisation, la pompe fonctionne grâce à l'énergie reçue par le chargeur sans fil.

Dans un mode de réalisation, la méthode de nettoyage comprend une phase de nettoyage au cours de laquelle :
- le dispositif reçoit de l'énergie par le chargeur pour alimenter notamment la pompe en énergie,
- la pompe est activée, de sorte que du liquide est aspirée par l'orifice de collecte.

La présente description concerne également un dispositif d'analyse d'urine comprenant :
- un boitier,
- un circuit fluidique logé à l'intérieur du boîtier, comprenant un orifice de collecte, un orifice de drainage et une pompe configurée pour faire circuler de l'urine dans le dispositif d'analyse d'urine,
- un système d'alimentation électrique configuré pour alimenter la pompe en énergie, le système d'alimentation électrique étant rechargeable par un chargeur à induction,
dans lequel le dispositif d'analyse d'urine est configuré pour sélectivement être :
en configuration d'analyse d'urine durant laquelle le circuit fluidique est configuré pour injecter de l'urine sur un réactif urinaire, ou
en configuration de nettoyage durant laquelle l'orifice de collecte est reliée à l'orifice de drainage,
dans lequel le circuit fluidique étant configuré pour, dans la configuration de nettoyage, faire circuler le fluide collecté par l'orifice de collecte directement vers l'orifice de drainage,
dans lequel le dispositif est configuré pour :
   - détecter que le système d'alimentation électrique est mis en contact avec un chargeur sans fil et,
   - en réponse à la détection, mettre le dispositif entre configuration de nettoyage.

### Présentation des figures

D'autres caractéristiques, détails et avantages apparaîtront à la lecture de la description détaillée ci-après, et à l'analyse des dessins annexés, sur lesquels :
- [FIG. 1] : la figure 1 présente une représentation schématique et simplifiée d'un dispositif d'analyse d'urine installé dans une cuvette de toilettes,
- [FIG. 2] : la figure 2 présente une vue éclatée du dispositif d'analyse d'urine, dans laquelle la station de mesure et la cartouche sont visibles,
- [FIG. 3] : la figure 3 présente une vue détaillée d'une cartouche selon un mode de réalisation en coopération avec un actionneur de la station de mesure,
- [FIG. 4] : la figure 4 présente une vue en coupe d'une cartouche et d'une station de mesure selon un mode de réalisation, à l'emplacement d'un analyseur optique de la station de mesure,
- [FIG. 5] : la figure 5 présente une vue de côté du dispositif d'analyse d'urine avec un bras de fixation,
- [FIG. 6] : la figure 6 présente une vue en perspective d'une station de nettoyage selon la présente description,
- [FIG. 7] : la figure 7 présente une vue en coupe verticale de la station de nettoyage, en configuration séparée (a) sans dispositif d'analyse d'urine inséré et en configuration opérationnelle (b) avec un dispositif d'analyse d'urine inséré,
- [FIG. 8] : la figure 8 présente une vue en coupe horizontale de la station de nettoyage, en configuration séparée (a) sans dispositif d'analyse d'urine inséré et en configuration opérationnelle (b) avec un dispositif d'analyse d'urine inséré,
- [FIG. 9] : la figure 9 présente une vie en perspective d'un couvercle de la station de nettoyage,
- [FIG. 10] : la figure 10 présente une vue en perspective d'un ensemble en position opérationnelle, avec le dispositif avec le bras de fixation inséré dans la station de nettoyage,
- [FIG. 11] : la figure 11 présente une vie en perspective d'un autre mode de réalisation du couvercle de la station de nettoyage,
- [FIG. 12] : la figure 12 présente une vue de face d'un ensemble en configuration séparée, avec le dispositif fixé au couvercle à l'écart de la station de nettoyage,
- [FIG. 13] : la figure 13 présente une vue en perspective d'un autre mode de réalisation de station de nettoyage (a) et une vue en coupe verticale de cette station de nettoyage (b),
- [FIG. 14] : la figure 14 présente une vue en perspective de l'ensemble en configuration opérationnelle, avec la station de nettoyage de la figure 13 et le dispositif d'analyse d'urine inséré dans la station de nettoyage,
- [FIG. 15] : la figure 15 présente une vue de côté d'un autre mode de réalisation d'un ensemble en configuration opérationnelle, dans la cuvette de toilettes,
- [FIG. 16] : la figure 16 présente schématiquement une vue des composants du dispositif selon un mode de réalisation de la description, ainsi que son écosystème.

### Description détaillée

La présente description présente différents exemples de dispositif d'analyse d'urine comprenant une station et une cartouche telle que divulguée dans les documents WO2021175909, WO2021175944, WO2023036805, WO2023036806, WO2023036808, WO2023036809.

Les paragraphes suivants expliquent le principe général d'un dispositif d'analyse d'urine, mais tous les détails des documents WO du paragraphe précédent sont applicables.

### Forme générale du boîtier

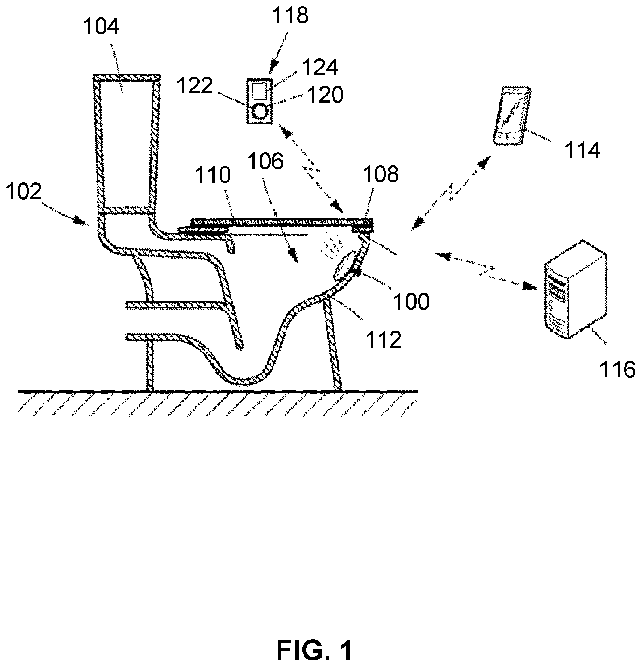

La figure 1 illustre schématiquement un dispositif d'analyse 100 (appelé aussi "dispositif 100" par la suite) pour l'analyse d'urine installé dans les toilettes 102. Les toilettes 102 comprennent généralement un réservoir d'eau 104, une cuvette 106, un siège 108 et un couvercle de siège 110. Le dispositif d'analyse 100 est configuré pour être placé entièrement dans la cuvette des toilettes. Par "dans la cuvette", on entend "placé dans le volume intérieur défini par la cuvette". Le dispositif d'analyse 100 est disposé de manière amovible dans les toilettes 102. Par exemple, le dispositif d'analyse 100 peut être facilement retiré des toilettes pour remplacer une cartouche, puis replacé dans les toilettes 102. Le dispositif d'analyse 100 est placé sur une paroi interne 112 de la cuvette 106 des toilettes. Le dispositif d'analyse 100 est placé de telle sorte qu'il se trouve généralement sous le jet d'urine d'un utilisateur, de sorte que lorsqu'un utilisateur urine (généralement en position assise), l'urine entre en contact avec le dispositif d'analyse 100. Le dispositif d'analyse 100 peut communiquer à distance avec une entité distante, telle que le smartphone 114 ou un serveur 116.

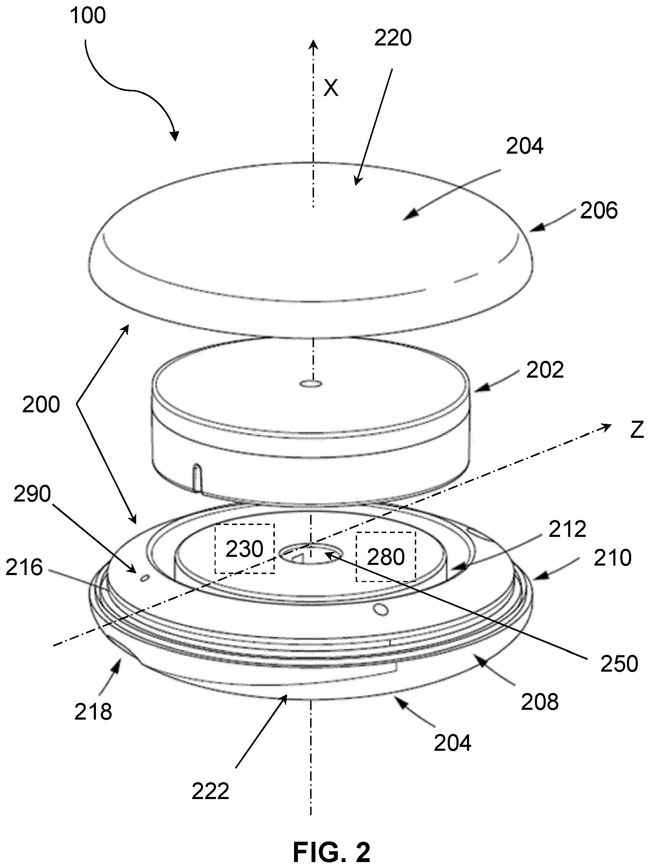

Comme l'illustre plus en détail la figure 2, le dispositif d'analyse 100 peut comprendre une station de mesure 200 et une cartouche 202, montée de manière amovible sur la station de mesure 200. La cartouche 202 comprend du réactif apte à réagir avec de l'urine (appelé aussi « réactif urinaire »). En variante, la station de mesure 200 comprend directement du réactif urinaire sans partie amovible. En variante, la station de mesure 200 peut se recharger en versant du réactif.

La station de mesure 200 peut comprendre un boîtier 204 qui peut comporter deux coques, notamment une coque avant 206 et une coque arrière 208. La coque avant 206 et la coque arrière 208 peuvent coopérer l'une avec l'autre par l'intermédiaire d'un mécanisme de fixation 216, dans un plan normal à l'axe X. La coque avant 206 et la coque arrière 208 peuvent être assemblées de manière réversible, par exemple par vissage ou par clipsage. Dans une variante, la coque avant 206 et la coque arrière 208 peuvent être assemblées de manière permanente, par exemple par collage, clipsage, magnétisation, ou soudage par ultrasons. Bien entendu, d'autres moyens de fixation peuvent être utilisés pour assembler les deux coques.

En particulier, comme illustré sur la figure 2, la coque avant 206 et la coque arrière 208 sont vissées l'une à l'autre. Ensuite, une partie interne de la coque avant 206 comprend un filetage. Le filetage de la coque avant 206 est destiné à coopérer avec un filetage complémentaire de la coque arrière 208. Cela permet de démonter facilement le boîtier 204 pour accéder à l'assemblage de test à l'intérieur du boîtier.

Un joint peut être présent entre la coque avant 206 et la coque arrière 208. Ainsi, le boîtier 204 est étanche. Seuls les orifices de collecte et de drainage relient l'extérieur et l'intérieur de la boîtier 204, comme décrit plus en détail ci-dessous.

Comme on peut le voir sur les figures, le boîtier 204 peut avoir une forme globale extérieure de galet circulaire. En d'autres termes, le boîtier 204 présente une forme sphéroïde. L'axe X est la ligne centrale du boîtier. Avantageusement, la coque avant 206 peut être sensiblement symétrique en rotation, ce qui donne un aspect aérodynamique au dispositif une fois installé. Le boîtier 204 sert de collecteur d'urine.

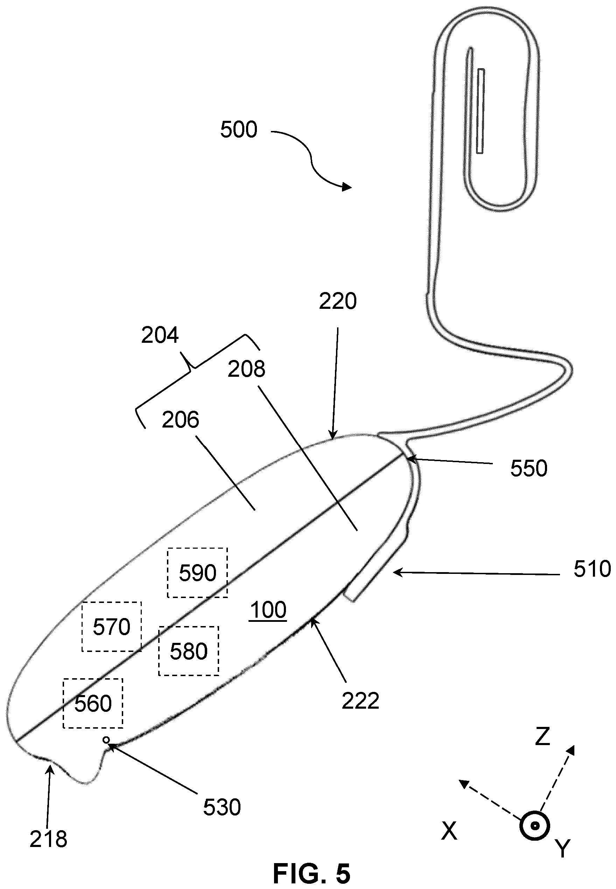

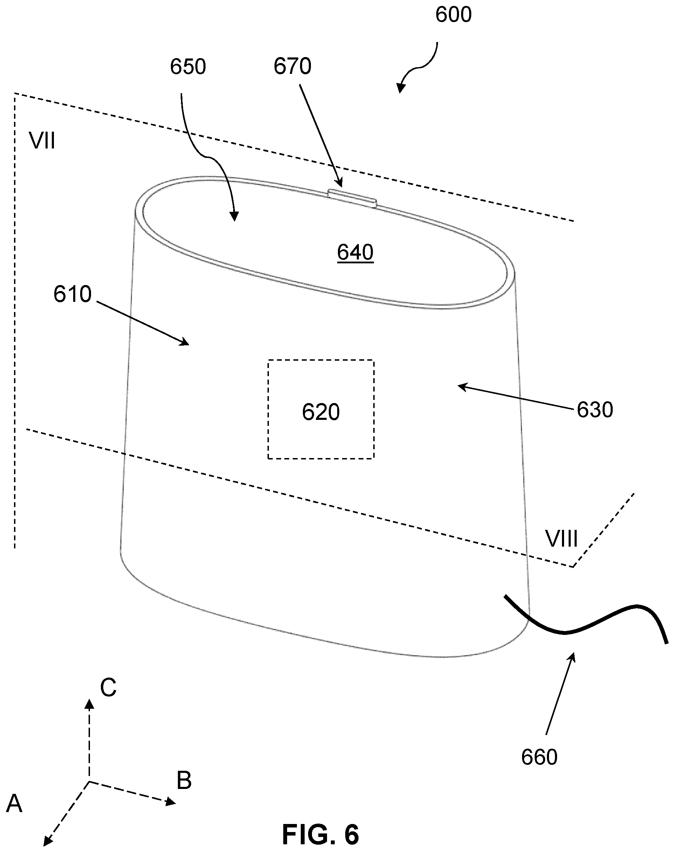

Le boîtier 204 comprend une face avant 220 pour recevoir un flux d'urine directement d'un utilisateur urinant sur les toilettes et une face arrière 222 opposée à la face avant 220. Comme illustré à la figure 2, la face avant 220 peut être disposée sur la coque avant 206 et la face arrière 222 peut être disposée sur la coque arrière 208. La face avant 220 est orientée vers l'intérieur de la cuvette 106. La face avant 220 est donc destinée à recevoir l'urine lorsque l'utilisateur urine en s'asseyant sur les toilettes 102. Comme le montrent les figures 5 et 6, la face arrière 222 fait face à la paroi intérieure 112 de la cuvette 106. Par la suite de la description, il est entendu par un objet faisant face à la paroi de la cuvette, un objet faisant face à la paroi de la cuvette la plus proche de l'objet en question, et non la paroi de la cuvette en vis-à-vis de l'autre côté du volume interne de la cuvette.

La face avant 220 et la face arrière 222 comportent chacune un bord incurvé 210. Les bords incurvés 210 respectifs de la face avant et de la face arrière se rejoignent au niveau d'une zone de jonction équatoriale. Ainsi, la surface extérieure du boîtier 204, constituée de la face avant 220 et de la face arrière 222, est définie par des lignes courbes et forme un objet généralement convexe.

La surface extérieure de la face avant 220 peut être lisse. En d'autres termes, la face avant 220 est dépourvue de crêtes ou de rainures. Ainsi, le flux d'urine entrant en contact avec la face avant 220 s'accroche et s'étale sur la face avant 220. La face avant 220 peut être sensiblement symétrique en rotation autour de l'axe X.

La surface extérieure du boîtier 204 peut également être blanche ou de couleur claire. La couleur de la surface extérieure peut être similaire à celle des toilettes, ce qui accroît la discrétion du dispositif.

Le boîtier 204 peut avoir un diamètre, mesuré dans la direction orthogonale à l'axe X, compris entre 50 mm et 150 mm. Le boîtier 204 peut avoir une épaisseur, mesurée dans la direction de l'axe X, comprise entre 15 mm et 50 mm. Ainsi, le boîtier 204 est suffisamment compact pour être entièrement logé dans la cuvette des toilettes. Le dispositif d'analyse d'urine 100 est discret. De plus, le boîtier 204 est suffisamment grand pour entrer systématiquement en contact avec l'urine reçue dans la cuvette. L'utilisateur peut alors uriner dans les toilettes sans se soucier du dispositif d'analyse d'urine, ou alternativement viser sommairement.

Selon un autre aspect, dans un mode de réalisation, le boîtier 204 présente un facteur de forme général tel que le rapport entre l'épaisseur et le diamètre est compris entre 0,2 et 0,5, et même de préférence entre 0,3 et 0,4. De telles proportions rappellent un galet naturel et donnent au dispositif un aspect apaisant.

De préférence, le boîtier 204 est constitué d'un matériau hydrophile. Par exemple, le matériau du boîtier 204 peut être : une céramique, un polyamide (PA), un silicone ou un polymère hydrophile. La surface extérieure du boîtier 204 peut également être traitée avec un traitement de surface hydrophile, par exemple acuWet^{®} d'Aculon, un polymère hydrophile, ou Pebax^{®} d'Arkema.

### Circuit fluidique

Un circuit fluidique 230 est présent à l'intérieur du boîtier 204 et configuré pour effectuer une analyse de l'urine collectée. La station de mesure 200 comprend un compartiment annulaire 212, situé à l'intérieur du boîtier 204, disposé autour d'un axe de rotation X. Le compartiment annulaire 212 est configuré pour recevoir au moins partiellement la cartouche 202 montée de manière rotative autour de l'axe de rotation X (une fois en position dans le compartiment annulaire 212). La cartouche 202 comprend une pluralité de supports de test qui comprennent chacun au moins un réactif urinaire, par exemple un réactif sec, la pluralité de supports de test étant disposée le long d'un cercle ou d'un arc de cercle autour de l'axe de rotation X. Dans un mode de réalisation, les supports de test sont des bandelettes de test. Les supports de test peuvent être enfermés, par exemple individuellement, dans une chambre.

Le compartiment annulaire 212 s'étend typiquement sur 360° et forme une gorge configurée pour recevoir au moins partiellement la cartouche 202.

La station de mesure 200 comprend un orifice de collecte 218, situé par exemple sur la coque arrière 208. L'orifice de collecte 218 est configurée pour collecter l'urine s'écoulant sur la surface du boîtier 204. La station de mesure 200 comprend également un orifice de drainage 530, visible sur la figure 5, configuré pour drainer le liquide collecté hors du dispositif 100.

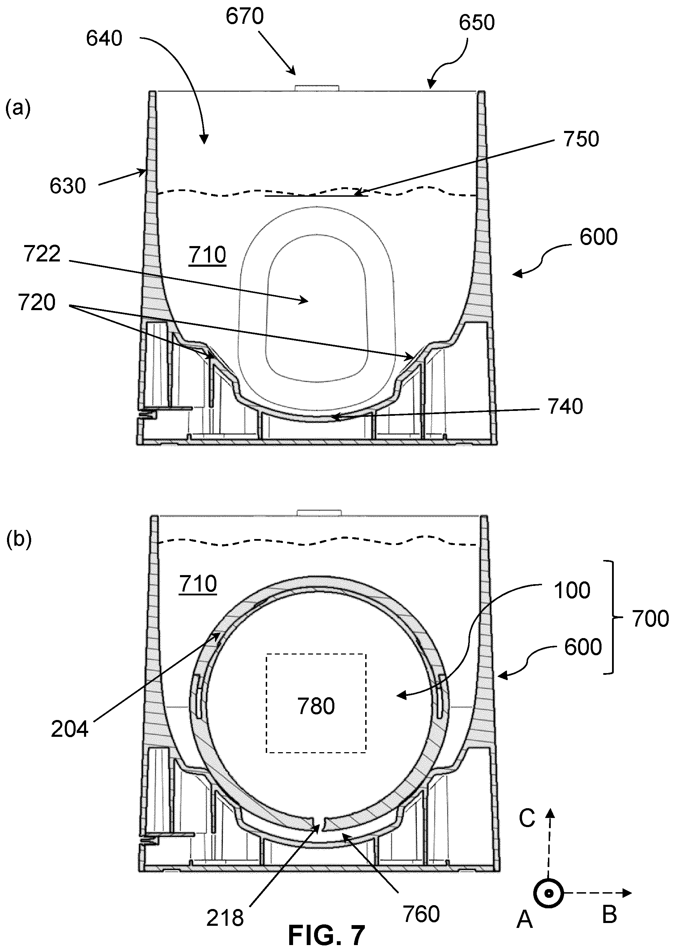

Le circuit fluidique 230 peut comprendre une pompe 780 (représentée schématiquement sur la figure 7), un injecteur et un analyseur 400. Dans une configuration de collecte, la pompe 780 aspire le liquide, notamment de l'urine, de l'orifice de collecte 218. Puis, dans une configuration d'injection, l'injecteur injecte l'urine sur un ou plusieurs supports de test de la cartouche et l'analyseur obtient certaines valeurs de propriétés (par exemple, des propriétés physiques/chimiques, telles que la couleur) des supports de test après qu'ils sont entrés en contact avec l'urine. Dans un cas, l'analyseur est un analyseur optique configuré pour analyser les propriétés optiques du support de test. L'injecteur et la cartouche peuvent se déplacer l'un par rapport à l'autre afin que l'injecteur puisse ouvrir (par exemple, percer) la chambre, par exemple à l'aide d'une aiguille ou d'un dispositif semblable à une aiguille.

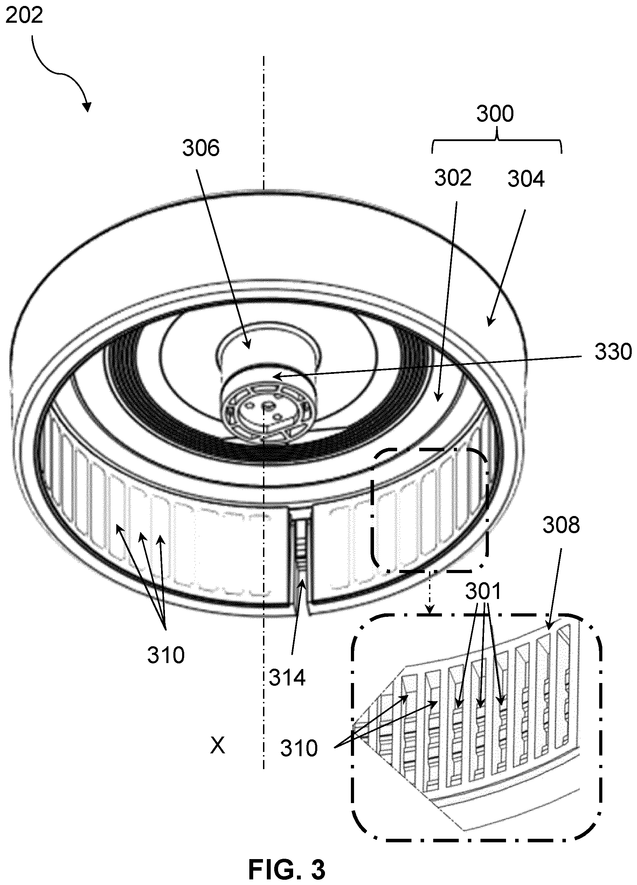

La figure 3 montre une vue éclatée de la cartouche 202. La cartouche 202 comprend au moins un support de test 301, notamment plusieurs supports de test 301 configurés pour recevoir l'urine de l'injecteur. Chaque support de test 301 contient un réactif urinaire qui réagit de manière spécifique au contact de l'urine. La cartouche 202 comprend un support rotatif 300, configuré pour être entraîné en rotation par la station de mesure 200. Dans le cadre d'une utilisation normale de la cartouche 202 et du dispositif 100, les supports de test 301 restent fixés au support rotatif et ne se déplacent pas par rapport à ce dernier.

Dans une réalisation, le support rotatif 300 a une forme de cylindre circulaire droit d'au moins 80% d'une forme de cylindre creux s'étendant annulairement autour d'un axe qui est, lorsque la cartouche 202 est montée dans la station de mesure 200, l'axe de rotation X. Chaque support de test 301 peut être une bandelette de test. Le support rotatif 300 peut comprendre une partie annulaire 302 et une partie cylindrique 304, qui s'étend à partir d'une extrémité radiale extérieure de la partie annulaire 302. La partie cylindrique 304, lorsqu'elle est utilisée, est logée à l'intérieur du compartiment annulaire 212. Les supports de test 301 sont positionnés le long de la portion cylindrique 304, de manière à pouvoir défiler sélectivement et/ou successivement devant l'injecteur et l'analyseur. Par exemple, les supports de test 301 font partie d'un support 308, qui comprend plusieurs chambres 310, séparées les unes des autres le long d'un périmètre autour de l'axe X. Au moins une bandelette de test est reçue dans une chambre 310.

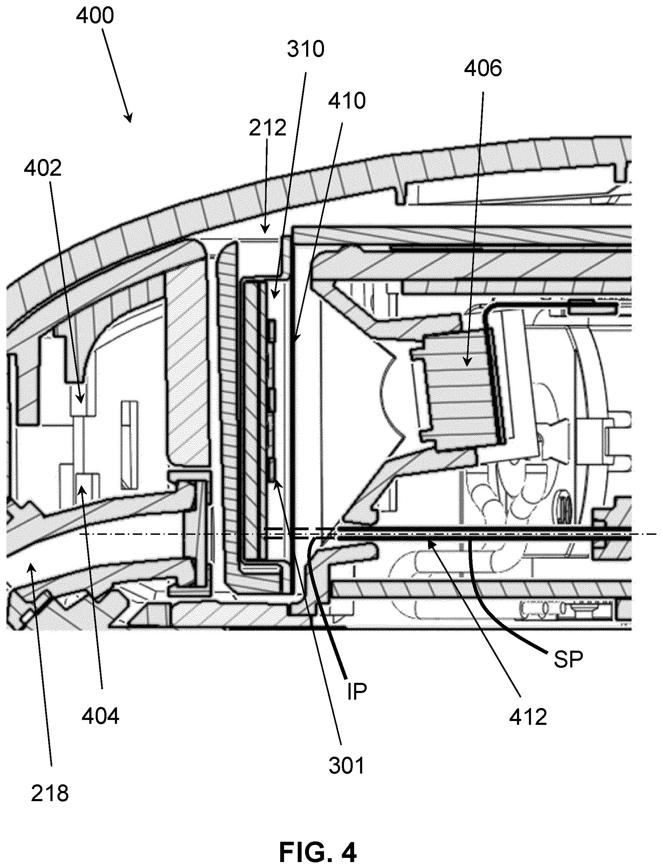

Les chambres 310 sont disposées les unes à côté des autres en forme de cylindre circulaire droit d'au moins 80 % du cercle. Pour permettre à la lumière de passer, le support 308 comprend au moins une ouverture 312 par chambre 310 (représentée dans le zoom supérieur gauche où le support rotatif est représenté comme transparent). Les chambres 310 sont toutes à égale distance de l'axe de rotation X, de sorte que l'injecteur peut injecter sélectivement l'urine une fois que la chambre souhaitée est positionnée à l'endroit voulu face à l'injecteur. L'injecteur peut se déplacer vers la chambre 310 et percer un opercule 410 fermant la chambre 310 (visible sur la figure 4). Dans un mode de réalisation, l'opercule 410 est transparent ou translucide pour permettre le passage de la lumière à l'intérieur de la chambre et/ou pour effectuer l'analyse optique du réactif, et ainsi permettre l'analyse du résultat de la réaction du réactif avec l'urine. Un orifice de vidange 314 est prévue dans le support rotatif 300 pour permettre l'évacuation de l'urine de l'injecteur vers l'extérieur du dispositif 100, via l'orifice de drainage 530 disposée sur le boîtier 204.

La partie annulaire 302 du support rotatif 300 reste à l'extérieur du compartiment annulaire 212 pour renforcer la partie cylindrique et/ou entraîner en rotation la cartouche 202. À cette fin, la partie annulaire 302 peut comprendre un accouplement mécanique 306, qui coopère avec un actionneur 330 de la station de mesure 200.

Les dimensions relatives à la cartouche 202 sont divulguées dans les documents WO'909, WO'933 et WO'80X. La dimension maximale du dispositif 100 transversalement à l'axe de rotation X est inférieure à 15 cm, voire inférieure à 10 cm. La dimension maximale du dispositif le long de l'axe de rotation X est inférieure à 5 cm.

La figure 4 montre plus en détail l'interaction entre la cartouche 202 et la station de mesure 200 lorsque ou après l'activation de l'injecteur. L'analyseur 400 comprend au moins une source lumineuse 402, 404 (par exemple, deux sources lumineuses; notamment quatre sources lumineuses) et au moins un capteur optique 406. La lumière va de la source lumineuse 602, 604 au capteur optique 606 en traversant la cartouche 202 et en particulier la partie cylindrique 304 et le support de test 301.

Dans un mode de réalisation, l'analyseur 400 est configuré pour mesurer l'absorbance d'une partie des supports de test 301 (notamment la ligne de test et/ou la ligne de contrôle d'une bandelette comme cela sera expliqué plus loin). L'absorbance est détectée par la source lumineuse (par exemple une LED) qui peut faire passer de la lumière à travers la bande, et le capteur optique qui reçoit le spectre avec une dizaine de longueurs d'onde.

Dans une variante, le capteur de lumière est une caméra capable de détecter un changement de couleur, notamment un changement d'intensité de couleur, d'une partie des supports de test 301 (notamment la ligne de test et/ou la ligne de contrôle d'une bandelette comme cela sera expliqué ci-dessous). La caméra peut détecter une couleur en valeurs RVB par exemple.

L'injecteur comprend une extrémité d'injection 412 (par exemple une aiguille), qui peut être déplacée entre une position d'attente SP et une position d'injection IP. Dans la position d'attente SP, l'extrémité d'injection 412 est à l'extérieur de la cartouche 202 (dans une position la plus intérieure radialement), de sorte que la cartouche 202 peut tourner librement dans le compartiment annulaire 212. Dans une position d'injection IP, l'extrémité d'injection 412 a percé l'opercule 410 pour accéder à l'intérieur de la chambre 310 et peut injecter un peu d'urine sur le réactif du support de test 301.

En position SP, l'injecteur est situé radialement à l'intérieur de la chambre annulaire. Ceci permet de maximiser le rayon du compartiment annulaire tout en minimisant la taille de la station de mesure 200.

Dans un mode de réalisation, l'injecteur peut être déplacé dans une configuration de collecte dans laquelle l'extrémité d'injection 412 est placée au niveau de l'orifice de collecte 218 pour permettre la collecte d'un liquide.

Dans un mode de réalisation, l'injecteur peut être déplacé dans une configuration de purge dans laquelle l'extrémité d'injection 412 est placée au niveau de l'orifice de drainage 530 pour permettre la purge du liquide.

Il est fait référence aux documents FR2410306, FR2410307 et FR2410308 (numéros de dépôt) plus de détail sur le fonctionnement de l'injecteur.

### Orifice de collecte

L'orifice de collecte 218 est configuré pour recevoir un liquide présent en vis-à-vis de l'orifice. L'orifice de collecte 218 est notamment configuré pour recevoir l'urine qui s'écoule par gravité sur la surface extérieure du boîtier 204 lorsque le dispositif est en position dans des toilettes. L'urine est collectée directement sur la face avant 220 et la face arrière 222 du boîtier 204. Comme cela sera expliqué plus en détail par la suite, l'orifice de collecte 218 permet en outre de collecter un liquide de nettoyage.

L'orifice de collecte 218 est une ouverture configurée pour collecter le liquide, de sorte que le liquide puisse pénétrer dans le dispositif d'analyse d'urine. L'orifice de collecte 218 est généralement circulaire, avec un diamètre de préférence compris entre 0,3 mm et 2 mm. Le diamètre de l'orifice de collecte peut être choisi pour maximiser le volume d'urine collecté sur la surface extérieure du boîtier 204.

Comme on peut le voir sur les figures, l'orifice de collecte 218 est situé sur la face arrière 222. Ainsi, l'orifice de collecte 218 fait face à la paroi interne 112 des toilettes lorsque le dispositif d'analyse d'urine 100 est positionné dans les toilettes. Cette position permet à l'orifice de collecte 218 d'être cachée à la vue de l'utilisateur par la face avant 220 du boîtier. La face avant 220 visible par l'utilisateur ressemble à un simple galet uniforme, comme déjà mentionné, sans points singuliers ni trous. Il convient également de noter que cette position empêche l'introduction de contaminants ou d'éléments qui pourraient obstruer le circuit fluidique 230.

L'orifice de collecte 218 est situé sur une partie inférieure de la face arrière 222. Par "sur une partie inférieure de la face arrière", on entend "sur le dernier quart de la face selon la direction Z à partir de l'extrémité inférieure du boîtier 204". L'extrémité inférieure fait face au fond de la cuvette 106 lorsque le boîtier 204 est positionné dans la cuvette. L'extrémité inférieure se situe à l'opposé du sommet 550. Cette position correspond à une position normale d'utilisation. Cette position permet à l'urine d'être collectée par gravité sur la majeure partie de la surface extérieure du boîtier 204.

En particulier, la distance séparant l'orifice de collecte 218 d'un bord inférieur du boîtier 204 est inférieure à 40 mm, de préférence inférieure à 20 mm. Comme illustré, selon un mode de réalisation particulier, l'orifice de collecte 218 est disposée à quelques millimètres au-dessus du bord inférieur du boîtier 204. En variante, l'orifice de collecte 218 peut se trouver sur le bord inférieur, le bord inférieur étant défini lorsque le dispositif 100 est placé pour être utilisé dans les toilettes.

L'orifice de collecte 218 peut être couvert par un filtre à mailles. Le filtre à mailles est par exemple de forme oblongue et recouvre l'orifice de collecte 218. L'ouverture moyenne des mailles du filtre est, par exemple, de 20 microns. Le filtre à maille empêche l'introduction de contaminants ou d'éléments susceptibles d'obstruer le circuit fluidique 230 et filtre l'urine reçue dans l'orifice de collecte 218. La maille du filtre peut être en métal.

### Système d'alimentation électrique

Le dispositif d'analyse d'urine 100 comprend un système d'alimentation électrique 280 configuré pour fournir de l'énergie au dispositif 100, et en particulier aux composants électroniques du dispositif 100. Le système d'alimentation électrique 280 peut être en outre configuré pour stocker de l'énergie.

Le système d'alimentation électrique 280 peut comprendre une batterie rechargeable. La batterie rechargeable est configurée pour stocker de l'énergie chimique et pour convertir cette énergie chimique en énergie électrique. La batterie est configurée pour être rechargée plusieurs fois, c'est-à-dire en inversant la réaction électrochimique pour recharger l'énergie chimique stockée dans la batterie. À cette fin, le dispositif 100 est configuré pour coopérer avec un chargeur de station tel qu'il sera expliqué ci-dessous. La batterie peut être par exemple une batterie Lithium Ion ou une batterie Lithium Polymère.

Comme cela sera expliqué plus en détail par la suite, le système d'alimentation électrique 280 peut être configuré pour alimenter les composants électroniques du dispositif 100, notamment la pompe 780, en parallèle de la recharge électrique de la batterie rechargeable par un chargeur.

Le dispositif d'analyse d'urine 100 peut comprendre un indicateur lumineux 290, notamment un indicateur de charge lumineux. L'indicateur lumineux 290 est configuré pour fournir une information visuelle relative à l'état de charge de la batterie. L'indicateur lumineux 290 est par exemple une LED configurée pour s'allumer en vert lorsque la batterie est à au moins 25% de sa charge, en orange lorsque la batterie est à au moins 5% de sa charge et en rouge lorsque la batterie est en dessous de 5%.

### Capteur de température

Le dispositif 100 peut comprendre un capteur de température 560 configuré pour détecter un changement de température au niveau du boitier 204. Le capteur de température 560 est monté dans le boitier 204, par exemple au niveau de l'orifice de collecte 218.

Par exemple, lorsque de l'urine à une température supérieure à 35°C ruisselle sur le boitier, le capteur de température 560 est configuré pour relever une augmentation soudaine de température. Le capteur de température 560 est alors configuré pour associer cette augmentation soudaine de température à la présence d'un jet d'urine sur le boitier 204, et par exemple déclencher une mise en route de la pompe 780.

Dans un autre exemple expliqué plus en détail par la suite, lorsque le dispositif 100 est placé dans un liquide de nettoyage présent dans un récipient, le capteur de température 560 est configuré pour détecter un changement de température sur le boitier, notamment une baisse de température, et envoyer un signal à une circuiterie de contrôle du dispositif 100.

### Capteur radar

Le dispositif 100 peut comprendre un capteur radar 570 configuré pour détecter la présence de corps à proximité du dispositif 100. A cet effet, le capteur radar 570 est configuré pour envoyer un signal radar et pour recevoir au moins un signal radar réfléchi, les réflexions étant provoquées par le ou les corps à proximité. Le traitement des signaux reçus permet de déterminer la présence et de caractériser ces corps. Par exemple, le capteur radar 570 est configuré pour détecter un jet d'urine et déterminer au moins une propriété relative à ce jet d'urine. En variante ou en complément, le capteur radar 570 est configuré pour détecter des parties génitales d'un utilisateur des toilettes afin notamment de l'identifier.

En variante ou complément, le capteur radar 570 est configuré pour détecter au moins une paroi à proximité du dispositif 100, notamment en vis-à-vis de la face avant 220 du boitier 204. Avantageusement, le capteur radar 570 est configuré pour détecter que le dispositif 100 est entouré d'une paroi présente à proximité de celui-ci. Comme cela sera expliqué par la suite, le capteur radar 570 permet donc de détecter que le dispositif 100 a été introduit dans un récipient.

Il est fait référence aux documents FR3140448 (numéro de publication), FR2403439 et FR2405542 (numéros de dépôt) qui décrivent l'implémentation d'un capteur radar dans le dispositif d'analyse d'urine.

### Capteur de liquide

Le dispositif 100 peut comprendre un capteur de liquide 580 configuré pour détecter une présence de liquide dans le circuit fluidique 230, notamment en réponse à l'activation de la pompe 780. Le capteur de liquide 580 est par exemple formé d'au moins deux sondes à deux positions séparées du circuit fluidique. Chaque sonde est notamment configurée pour identifier l'état (liquide, gazeux ou fluide mixte liquide/gaz) du fluide circulant devant la sonde. Le capteur de liquide 580 peut en outre être configuré pour déterminer le débit du fluide pompé par l'orifice de collecte 218.

Il est fait référence au document WO2022184984 qui décrit plus en détail le fonctionnement d'un tel capteur de liquide 580.

### Fixation

Le boîtier 204 est destiné à être placé sur la paroi interne 112 de la cuvette 106. Le boîtier 204 est fixé par un bras de fixation 500. Le bras de fixation 500 est configuré pour fixer le dispositif d'analyse d'urine 100 dans la cuvette 106 des toilettes. Le bras de fixation 500 est typiquement réalisé en un seul matériau, par exemple du plastique, par moulage.

Le dispositif d'analyse 100 comprend un élément d'attache 510 disposé sur la face arrière 222 du boîtier. L'élément d'attache 510 est configuré pour coopérer avec un bras de fixation 500.

L'élément d'attache 510 est disposé au niveau d'un plan médian XZ du boîtier 204. En d'autres termes, l'élément d'attache 510 est disposé au niveau d'un plan de symétrie vertical du boîtier 204.

Comme on peut le voir sur les figures, l'élément d'attache 510 peut être disposé sur la partie supérieure du boîtier 204. Par "sur la partie supérieure du boîtier", on entend "sur le premier quart le long de la direction Z à partir du sommet du boîtier 204". En particulier, l'élément d'attache 510 est disposé entre 15% et 25% de la face arrière 222 en partant d'un sommet 550 du boîtier 204.

L'élément d'attache 510 peut être un ergot. L'ergot fait saillie hors de la face arrière 222 du boîtier 204.

En variante, l'élément d'attache 510 est un aimant apte à coopérer avec un autre aimant placé sur le bras de fixation 500.

En variante, l'élément d'attache 510 comprend une partie adhésive apte à coopérer avec le bras de fixation 500.

Selon un mode de réalisation, le bras de fixation 500 peut être amovible. Autrement dit, l'utilisateur peut séparer le bras de fixation 500 de l'élément d'attache 510 et donc du boitier. Le bras de fixation 500 peut être ainsi aisément remplacé et une autre forme de bras de fixation peut être par exemple utilisée.

Le bras de fixation 500 peut être flexible. Autrement dit, le bras de fixation 500 peut être déformé par l'utilisateur ou par la gravité du dispositif 100 placé dans les toilettes. Ceci permet ainsi une meilleure adaptation du bras de fixation aux différentes formes de toilettes et assure que le boîtier 204 touche la paroi interne 112 des toilettes.

Le bras de fixation 500 peut s'étendre selon une hauteur selon l'axe Z comprise entre 5 cm et 15 cm, dans une configuration de repos (c'est-à-dire ni étiré ni comprimé). Le bras de fixation 500 présente par exemple une largeur selon l'axe Y comprise entre 1 cm et 2 cm. Le bras de fixation 500 présente par exemple une épaisseur comprise entre 1 mm et 2 mm.

L'orifice de collecte 218 et l'élément d'attache 510 (ou le bras de fixation 500) peuvent être disposés de part et d'autre du dispositif d'analyse d'urine 100, de sorte que lorsque le dispositif 100 est tenu via l'élément d'attache 510, ce dernier se trouve en partie haute et l'orifice de collecte 218 se trouve en partie basse (par rapport à la verticale définie par la gravité).

### STATION

Une station de nettoyage 600 pour dispositif d'analyse d'urine 100 est représentée sur la figure 6. La station de nettoyage 600 est configurée pour recevoir le dispositif d'analyse d'urine 100. La station de nettoyage 600 forme, avec le dispositif d'analyse d'urine 100, un ensemble 700.

La station de nettoyage 600 comprend un récipient 610 et un chargeur 620. Le récipient 610 est configuré pour recevoir un liquide de nettoyage 710. Le récipient 610 est en outre dimensionné pour recevoir au moins en partie le dispositif d'analyse d'urine 100, de sorte que le dispositif d'analyse d'urine 100 soit au contact du liquide de nettoyage 710 lorsqu'il est inséré dans le récipient 610.

Le chargeur 620 est configuré pour alimenter en énergie le dispositif d'analyse d'urine 100 lorsque le dispositif d'analyse d'urine 100 est reçu au moins en partie dans le récipient 610.

L'ensemble 700 est configuré pour passer d'une configuration opérationnelle à une configuration séparée, et vice et versa.

Dans la configuration opérationnelle, le dispositif d'analyse d'urine 100 est reçu dans le récipient 610, comme représenté sur les figures 7 (b), 8 (b), 10, 14 et 15.

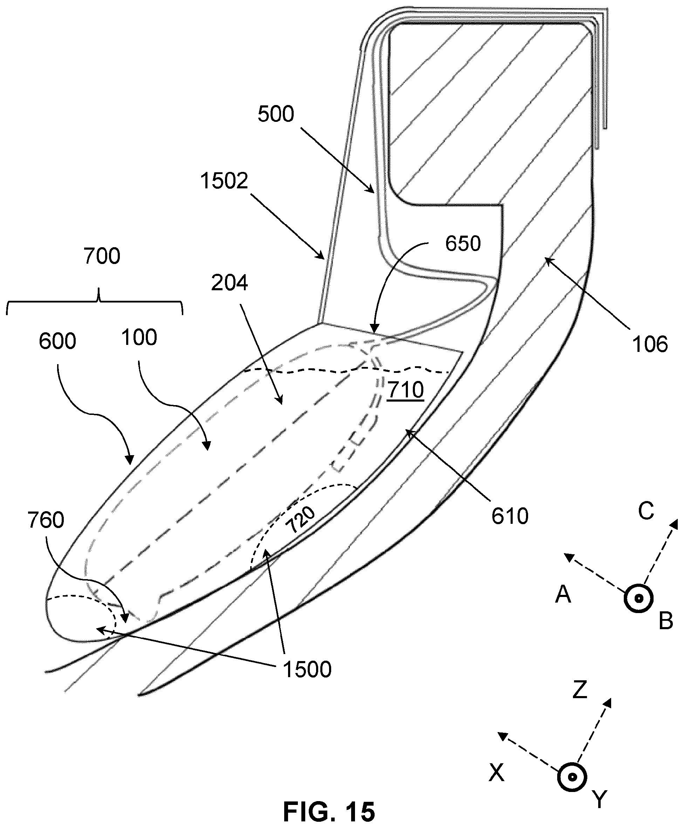

Dans un mode de réalisation, représenté sur les figures 7 à 14, le dispositif d'analyse d'urine 100 est à l'écart de la cuvette 106 de toilettes dans la configuration opérationnelle. En variante, représentée sur la figure 15, le dispositif d'analyse d'urine 100 est en position dans la cuvette 106 en configuration opérationnelle.

Dans la configuration séparée, le dispositif d'analyse d'urine 100 est à l'écart du récipient 610. En particulier, le dispositif 100 est configuré pour être placé entièrement dans la cuvette 106 de toilettes dans la configuration séparée, comme représenté sur la figure 1.

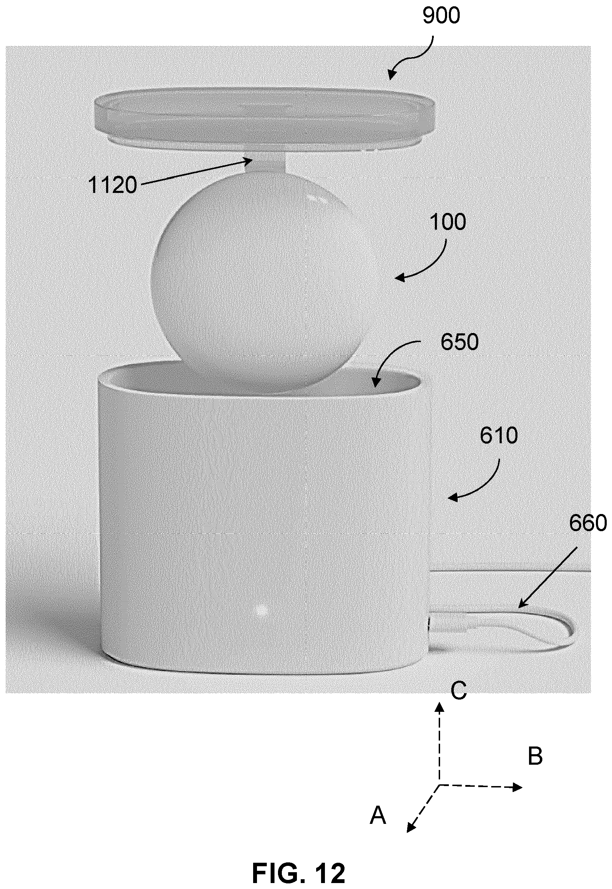

Sur la figure 12, le dispositif 100 est en transition entre la configuration opérationnelle et la configuration séparée.

Ainsi, le récipient 610 est configuré pour recevoir de manière amovible le dispositif d'analyse d'urine 100, entre la configuration séparée et la configuration opérationnelle de l'ensemble 700. Dans les modes de réalisations représentés sur les figures 7 à 14, le dispositif 100 est inséré dans la station 200 pour passer dans la configuration opérationnelle.

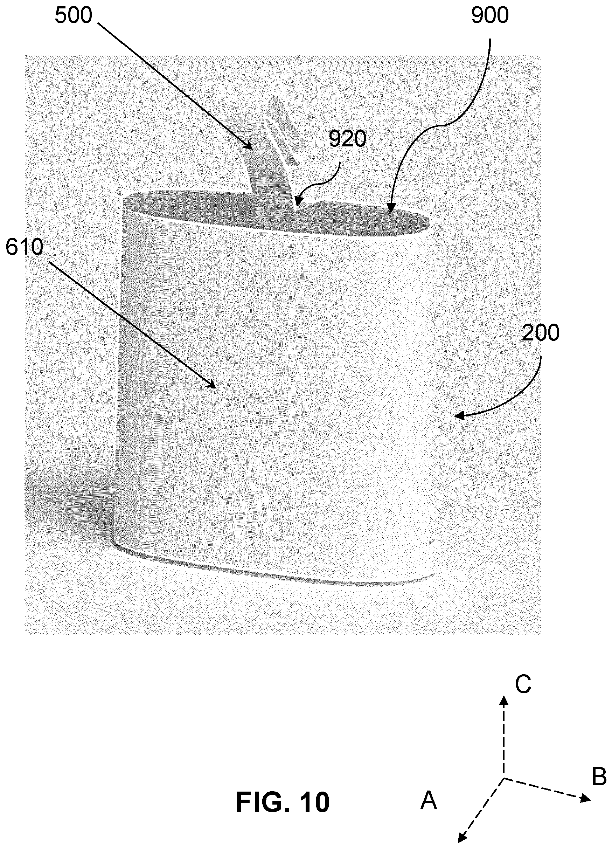

Dans le mode de réalisation sur la figure 15, la station 200 est insérée autour du dispositif 100 dans la cuvette 106 pour passer dans la configuration opérationnelle. Le bras de fixation 500 fait alors saillie hors du récipient 610. La station 200 peut alors comprendre un moyen de fixation 1502 du récipient 610 à la cuvette 106, notamment un ou plusieurs bras ou un aimant. En variante, le récipient 610 repose directement sur le boitier 204 et sur le bras de fixation 500 du dispositif, notamment via le couvercle 900 de manière similaire au mode de réalisation de la figure 10. La station 200 peut alors ne pas comprendre de moyen de fixation 1502 à la cuvette 106.

La station de nettoyage 600 a pour rôle de nettoyer le dispositif d'analyse d'urine 100. Ce nettoyage comprend principalement le nettoyage du circuit fluidique 230. A cette fin, le nettoyage s'effectue en introduisant du liquide nettoyage 710 dans le circuit fluidique 230 via l'orifice de collecte 218 par activation de la pompe 780.

### Forme du récipient

Un référentiel direction ABC, avec trois axes A, B, C orthogonaux deux à deux, est utilisé pour décrire la forme du récipient.

Le récipient 610 présente une forme creuse. En particulier, le récipient 610 comprend une paroi 630 qui définit un volume interne 640. Le volume interne 640 est accessible via une ouverture 650. Le récipient 610 comprend un fond 740, à l'opposé de l'ouverture 650, visible en figure 7.

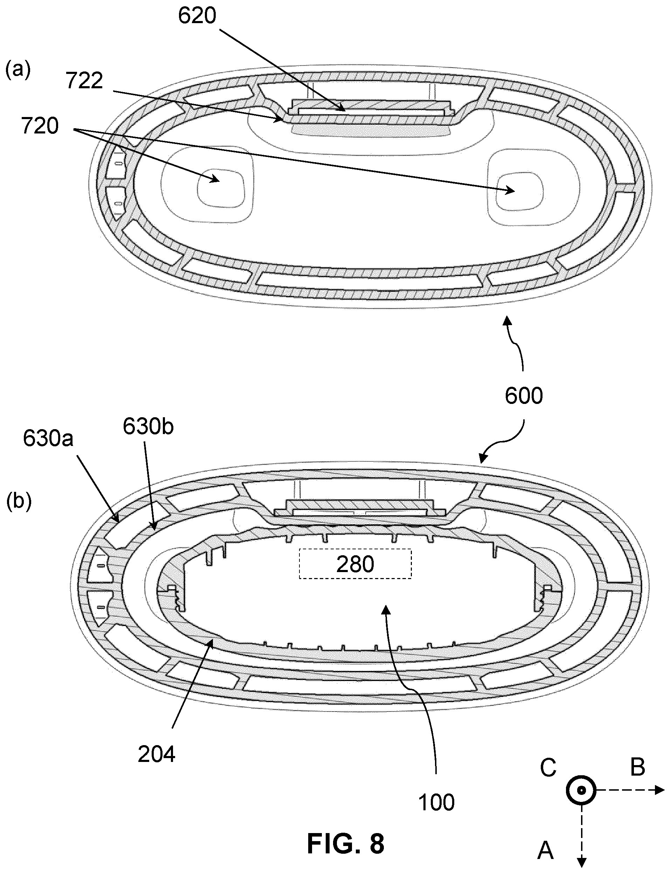

Dans un mode de réalisation, visible sur la figure 8, la paroi 630 peut être une double cloison afin de rigidifier le récipient 610, avec une cloison externe 630a à l'extérieur du récipient 610 et une cloison interne 630b à l'intérieur du récipient 610 définissant le volume interne 640. En variante non représentée, la paroi 630 peut être constituée d'une unique cloison.

Dans un mode de réalisation, comme représenté sur les figures 6 à 10, le récipient 610 présente une forme de cône tronqué. Notamment la base du cône (donc la partie au contact du support sur lequel la station de nettoyage 600 est posée) présente la section la plus importante. Cette forme permet d'améliorer la stabilité de la station de nettoyage 600, notamment lorsque le volume interne 640 est empli de liquide. La base du récipient est par exemple un ovale ou plus généralement une forme convexe arrondie (donc le rond, l'ovoïde, l'ellipse, etc.). En variante non représentée, la base peut être de toute forme géométrique telle qu'un carré, un rectangle, un triangle etc.

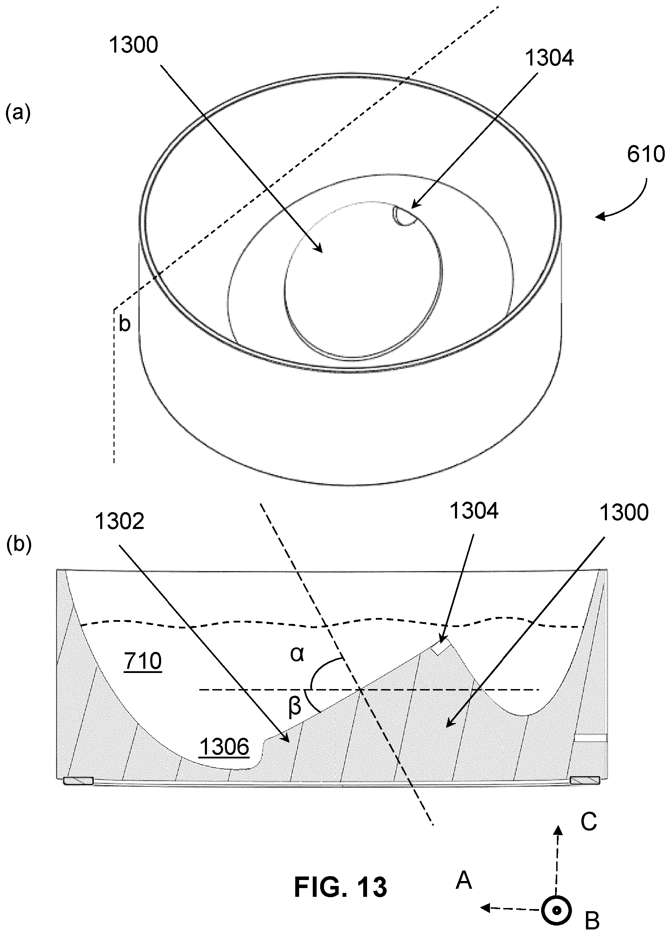

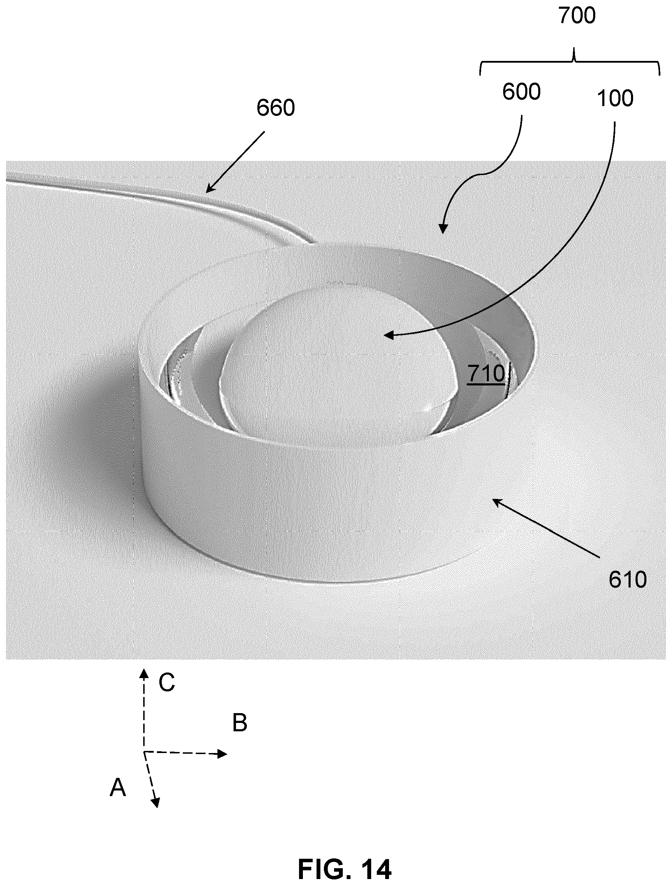

Dans un autre mode de réalisation, comme représenté sur les figures 12 à 14, le récipient 610 peut présenter une forme cylindrique droite. La base du récipient est par exemple ovale, comme représenté sur la figure 12. En variante, la base du récipient est par exemple ronde, comme représenté sur les figures 13 et 14. En variante non représentée, la base peut être de toute forme géométrique tel un carré, un rectangle, un triangle etc.

Dans un autre mode de réalisation, comme représenté sur la figure 15, le récipient 610 est de forme complémentaire à la surface interne de la cuvette 106. En particulier, le récipient 610 peut présenter une forme ovoïdale.

Dans un mode de réalisation, comme représenté sur les figures 6 à 12 et 15, le récipient 610 est plus haut que large. Autrement dit, le récipient s'étend sur une hauteur selon l'axe C supérieure aux dimensions transversales, selon les axes A ou B. En particulier, la hauteur du récipient selon l'axe C est par exemple comprise entre 10 cm et 30 cm. La longueur du récipient selon l'axe B est par exemple comprise entre 5 cm et 20 cm. La largeur du récipient selon l'axe A est par exemple comprise entre 3 cm et 10 cm.

En variante, comme représenté sur les figures 13 et 14, le récipient 610 est plus large que haut. Autrement dit, le récipient s'étend sur une hauteur selon l'axe C inférieure aux dimensions transversales, selon les axes A ou B. Cette forme permet d'améliorer la stabilité de la station de nettoyage 600, notamment lorsque le volume interne 640 est empli de liquide. En particulier, la hauteur du récipient selon l'axe C est par exemple comprise entre 3 cm et 10 cm. La longueur du récipient selon l'axe B est par exemple comprise entre 10 cm et 20 cm. La largeur du récipient selon l'axe A est par exemple comprise entre 10 cm et 20 cm.

Dans un mode de réalisation, le dispositif d'analyse d'urine 100 présente une dimension de moindre longueur selon l'axe A. Le récipient 610 est configuré pour recevoir le dispositif 100 de sorte que l'axe A s'étende horizontalement, parallèlement à l'axe B du récipient. Le dispositif 100 est donc reçu verticalement selon l'axe C dans le récipient 610.

En particulier, la largeur du récipient 610 selon l'axe A est sensiblement égale à la dimension de moindre longueur du dispositif 100. Seul un jeu fonctionnel est présent entre la paroi 630 et le boitier 204 du dispositif 100 pour permettre l'insertion du dispositif 100 dans le récipient 610. Ainsi, le dispositif 100 est soutenu latéralement par la paroi 630 afin de caler le dispositif 100 dans le récipient.

En variante, comme représenté sur la figure 14, le récipient 610 est configuré pour recevoir le dispositif 100 de sorte que l'axe A s'étende selon une direction formant un angle α compris entre 20° et 70°, par exemple d'environ 45° avec la direction horizontale X. Autrement dit, le dispositif 100 est reçu de manière inclinée dans le récipient 610, avec l'orifice de collecte 218 placé en bas.

Le récipient 610 est conçu pour former un volume de dégagement 760 autour de l'orifice de collecte 218. Ce volume de dégagement 760 se définit notamment par le fait que l'orifice de collecte 218 est à l'écart du la paroi 630 et/ou du fond 740, afin d'avoir un volume de liquide de nettoyage suffisant dans le voisinage immédiat de l'orifice de collecte 218 pour son aspiration.

Le récipient 610 peut comprendre au moins une saillie 720, 722, 1300, 1500 configurée pour supporter le dispositif d'analyse d'urine 100 dans la configuration opérationnelle.

Dans un mode de réalisation représenté sur les figures 7 et 8 notamment, l'au moins une saillie 720 forme un ber (ou berceau) pour tenir en place le dispositif d'analyse d'urine 100 dans la station de nettoyage en configuration opérationnelle. Chaque ber est notamment conçu pour définir le volume de dégagement 760 dans le voisinage immédiat de l'orifice de collecte 218.

En particulier, au moins une saillie 720 est arrangée en partie basse du récipient 610. Autrement dit, la saillie 720 est disposée sur le fond 740 ou dans le premier quart selon l'axe C en partant du fond 740 du récipient.

Avantageusement, le dispositif 100 est uniquement soutenue par la ou les saillies 720 dans le récipient 610. Autrement dit, le boitier 204 n'est en contact qu'avec les saillies 720 et n'est pas en contact avec le reste de la paroi 630.

Dans un mode de réalisation visible sur la figure 7, le récipient 610 comprend au moins deux saillies 720 disposées à proximité du fond 740 du récipient 610. Selon un mode de réalisation, les deux saillies 720 sont disposées symétriquement de part et d'autre du récipient selon l'axe B. Comme cela sera expliqué plus en détail par la suite, les saillies 720 permettent de laisser libre l'orifice de collecte 218 et ne pas l'obstruer pour permettre le passage du liquide de nettoyage 710.En particulier, les saillies 720 contribuent à définir le volume de dégagement 760.

En variante ou en complément, le récipient 610 comprend au moins une saillie 722 latérale faisant saillie sensiblement horizontalement de la paroi 630. La ou les saillies 720 latérales permettent de caler le dispositif 100 latéralement dans le récipient 610 et de le maintenir en position.

Dans un mode de réalisation représenté sur la figure 13, le récipient 610 comprend au moins une saillie 1300 se présentant sous la forme d'un promontoire configuré pour supporter le dispositif 100. En particulier, la saillie 1300 comprend une surface de réception 1302 configurée pour être en contact avec l'une des deux faces 220, 222 du boitier 204 afin de supporter le dispositif 100. La surface de réception 1302 s'étend selon un plan formant un angle β avec la direction horizontale compris entre 0° et 70°. La surface de réception 1302 est avantageusement de forme complémentaire à la face 220, 222 du boitier 204 avec laquelle elle est contact. En particulier, la saillie 720 peut comprendre au moins un élément de maintien 1304 configuré pour coopérer avec la face 220, 222 du boitier de sorte à la maintenir en position. Comme représenté sur la figure 13, l'élément de maintien 1304 est par exemple une encoche de forme complémentaire avec au moins une partie du dispositif 100, notamment avec l'élément d'attache 510 du boitier 204.

Similairement au récipient 610 de la figure 7, le récipient 610 de la figure 13 définit un volume de dégagement 1306 au sein duquel l'orifice de collecte 218 se trouve lorsque le dispositif 100 est en position opérationnelle.

Dans les modes de réalisation des figures 7 et 13, du fait du positionnement de l'orifice de collecte 218 sur le boitier 204 et du positionnement du dispositif 100 dans la station de nettoyage 600, le volume de dégagement 1306 se trouve au niveau du fond du récipient 610.

### Couvercle

La station de nettoyage 600 peut comprendre un couvercle 900 configuré pour fermer au moins en partie le volume interne 640 du récipient 610. Autrement dit, le couvercle 900 est configuré pour obstruer au moins partiellement l'ouverture 650 du volume interne 640.

Le couvercle 900 est configuré pour passer d'une configuration ouverte à une configuration fermée, et vice et versa. Dans la configuration ouverte, le couvercle 900 est à l'écart de l'ouverture 650. La configuration ouverte permet d'insérer ou retirer aisément le dispositif 100 du récipient 610. Dans la configuration fermée, le couvercle 900 coopère mécaniquement avec le récipient 610 pour fermer au moins partiellement l'ouverture 650. La configuration fermée permet de maintenir le dispositif 100 à l'intérieur du récipient 610.

Le couvercle 900 est par exemple formé d'un matériau plastique. Le couvercle peut être au moins partiellement transparent ou translucide, de sorte que l'utilisateur puisse voir au moins partiellement à travers le dispositif 100 à travers le couvercle 900 lorsque l'ensemble est en configuration opérationnelle et le couvercle 900 en configuration fermée.

Le couvercle 900 peut présenter une forme sensiblement complémentaire à l'ouverture 650. Dans un mode de réalisation, le couvercle 900 peut s'enclencher par clipsage au niveau de l'ouverture 650. En variante ou en complément, le couvercle 900 peut être aimanté au récipient 610. En variante ou en complément, le couvercle 900 peut être lié par une charnière au récipient 610.

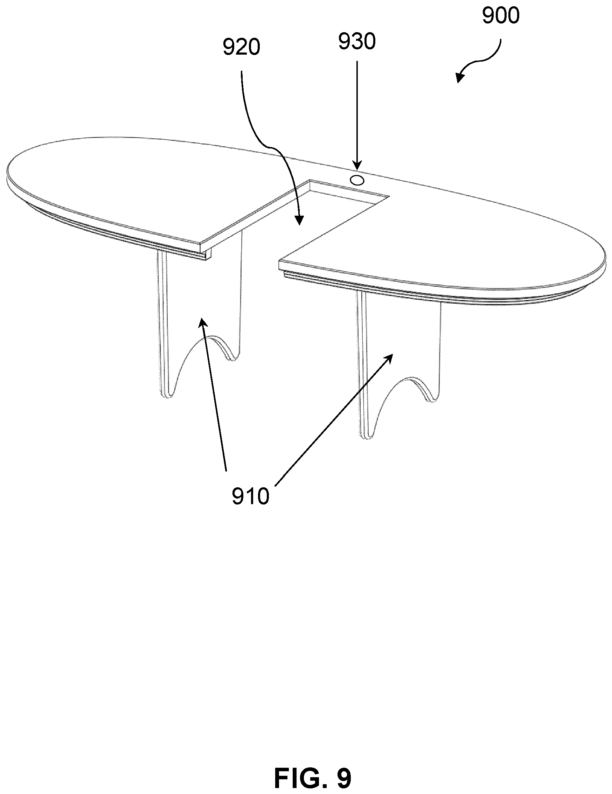

En référence à la figure 9, le couvercle 900 peut comprendre au moins une cale 910 faisant saillie du couvercle 900 vers le volume interne 640 du récipient 610 dans la configuration fermée. Chaque cale 910 est configurée pour maintenir le dispositif 100 en position dans le récipient 610. En particulier, chaque cale 910 est dimensionnée de sorte à être en contact avec le boitier 204 dans la configuration fermée du couvercle 900 (un léger jeu fonctionnel peut être prévu). Chaque cale 910 peut comprend une extrémité de forme complémentaire avec le boitier 204. En configuration opérationnelle de l'ensemble et dans la configuration fermée du couvercle, le dispositif 100 est alors maintenu en position par l'au moins une saillie 720 et l'au moins une cale 910. Comme visible sur la figure 9, le couvercle peut comprendre deux cales 910.

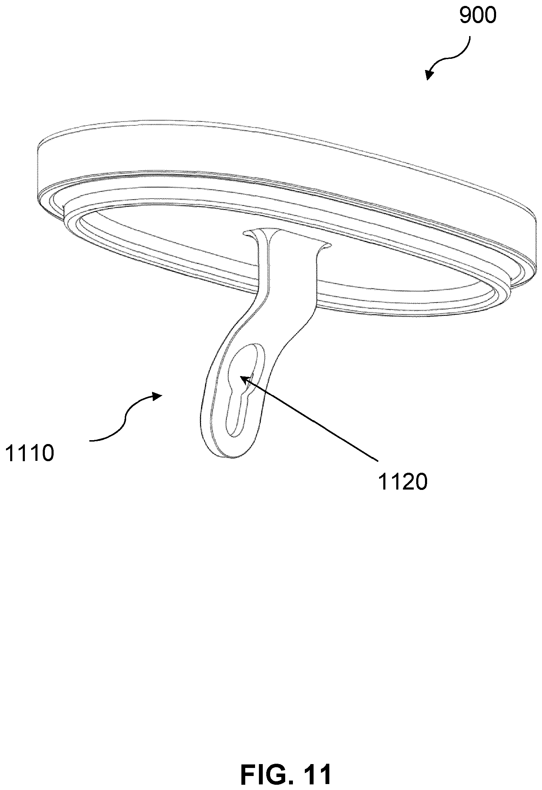

En variante représentée sur la figure 11, le couvercle 900 peut comprend un crochet 1110 configuré pour coopérer avec l'élément d'attache 510 du dispositif d'analyse d'urine 100. Le crochet 1110 fait saillie en direction du volume interne 640 en configuration fermée du couvercle 900. Lorsque l'élément d'attache 510 est un ergot, le crochet peut comprendre une ouverture 1120 apte à recevoir l'ergot. En variante, lorsque l'élément d'attache 510 est un aimant, le crochet 110 peut comprendre un aimant complémentaire pour se lier à l'aimant du dispositif 100. Comme représenté sur la figure 12, le dispositif 100 peut être ainsi lié au couvercle 900 dans la configuration ouverte du couvercle et dans la configuration séparée de l'ensemble. Le dispositif 100 peut être alors aisément être inséré dans le récipient 610 en même temps que le couvercle 900 est placé sur l'ouverture 650 de sorte que le couvercle 900 passe en configuration fermé en même temps que l'ensemble passe dans la configuration opérationnelle.

Dans un mode de réalisation, comme représenté sur la figure 9, le couvercle 900 peut comprendre au moins une ouverture traversante 920. L'ouverture traversante 920 est apte à être traversée par un bras de fixation 500 du dispositif d'analyse d'urine 100, lorsque l'ensemble 700 est en configuration opérationnelle et lorsque le couvercle 900 est en configuration fermée, comme visible sur la figure 10. Le bras de fixation 500 fait ainsi saillie au moins en partie du récipient 610 dans la configuration opérationnelle. Le récipient 610 peut comprendre un plot 670 faisant saillie hors du rebord de l'ouverture 650. L'ouverture traversante 920 est de forme complémentaire au plot 670. Le plot 670 permet notamment d'orienter l'utilisateur dans le sens où refermer le couvercle 900 sur l'ouverture 650.

L'ouverture traversante 920 permet de ne pas avoir à enlever le bras de fixation 500 lorsque le dispositif 100 est placé dans le récipient 610. L'utilisateur peut ainsi manipuler facilement le dispositif 100 en saisissant le bras pour l'introduire ou le retirer du récipient 610. Le bras de fixation 500, ne recevant globalement pas d'urine dans la cuvette, peut être mieux adapté pour une préhension par un utilisateur. Ainsi, l'utilisateur peut déplacer le dispositif 100 des toilettes à la station de nettoyage sans toucher le boîtier 204 ayant reçu de l'urine. En outre, la partie proximale du bras de fixation 500 (celle proche du boîtier 204) peut être ainsi lavée dans la station de nettoyage 600.

En variante, comme représenté sur la figure 11, le couvercle 900 ne comprend pas d'ouverture. Autrement dit, le couvercle 900 est dimensionné pour obstruer entièrement l'ouverture 650 du récipient 610 dans la configuration fermée.

En variante ou en complément, le couvercle 900 peut comprendre une zone de moindre épaisseur 930. La zone de moindre épaisseur 930 permet de rendre visible l'indicateur lumineux 290 du dispositif 100, notamment l'indicateur de charge de la batterie 280 du dispositif 100, par l'utilisateur à travers le couvercle 900. La zone de moindre épaisseur 930 présente par exemple une épaisseur inférieure à 2 mm. La zone de moindre épaisseur 930 présente par exemple la forme d'un rond de diamètre inférieur à 1 cm.

### FONCTION NETTOYAGE - côté station

Comme expliqué ci-dessus, le récipient 610 est dimensionné pour recevoir au moins en partie le dispositif d'analyse d'urine 100, de sorte que le dispositif d'analyse d'urine 100 soit au contact du liquide de nettoyage 710 lorsqu'il est inséré dans le récipient 610.

En particulier, comme visible sur la figure 7 (b), le récipient 610 et l'au moins une saillie 720, 722, 1300, 1500 sont conçus pour que l'orifice de collecte 218 soit immergé dans le liquide de nettoyage 710 en configuration opérationnelle.

En particulier, le récipient 610 est en outre dimensionné, avec notamment l'au moins une saille 720, 722, 1300, pour définir le volume de dégagement 760. Ce volume se définit notamment par le fait que l'orifice de collecte 218 est à l'écart de la paroi 630 et/ou du fond 740 du récipient 610. Par « à l'écart », il est signifié au moins 3 mm, notamment au moins 5 mm, entre l'orifice de collecte 218 et le plus éloigné parmi la paroi 630 ou le fond 740. Comme cela sera expliqué ci-dessous, cela permet ainsi le pompage non entravé du fluide de nettoyage 710 par le dispositif 100 afin de permettre son nettoyage de l'intérieur, en particulier le nettoyage du circuit fluidique 230.

Dans un mode de réalisation, le récipient 610 est dimensionné de sorte que le boitier 204 soit complétement immergé sous le liquide de nettoyage 710 en configuration opérationnelle. L'intégralité de l'extérieur du boitier 204 peut être ainsi nettoyé de manière passive par son contact avec le liquide de nettoyage 710. En effet, le boitier 204 est régulièrement au contact d'urine, ce qui peut le salir et poser des problèmes d'hygiène. La station de nettoyage 600 permet donc un nettoyage au moins partiel de l'extérieur du boitier 204 du dispositif 100 de manière passive.

Dans un mode de réalisation, la station de nettoyage 600 est dépourvue de moyens de circulation du liquide de nettoyage 710 dans le récipient 610. La station de nettoyage 600 est ainsi un système passif pour le nettoyage, le dispositif 100 étant l'élément faisant circuler activement le liquide de nettoyage 710. La mise en mouvement du fluide par le dispositif 100 favorise en outre le nettoyage de l'extérieur du boîtier 204.

En variante ou en complément, la station de nettoyage 600 peut comprendre une pompe configurée pour faire circuler le liquide de nettoyage 710 dans le récipient 610 et injecter le fluide dans le dispositif 100. La pompe peut aussi simplement générer une circulation de liquide de nettoyage dans le récipient 610 pour favoriser le nettoyage du boîtier 204.

Pour que l'utilisateur soit assuré d'avoir mis une quantité suffisante de liquide nettoyage, le récipient 610 peut comprendre une jauge 750 indiquant le volume recommandé de liquide de nettoyage 710 à verser dans le récipient 610 lorsque le dispositif 100 n'est pas inséré dans le récipient 610. La jauge 750 est par exemple une marque horizontale placée sur la paroi 630, notamment à l'intérieur du volume interne 640.

Le liquide de nettoyage 710 est notamment du détergent, par exemple du Tergazyme ^{®}. En variante, le liquide de nettoyage est de l'eau. Le liquide nettoyage peut être généré en mélangeant une pastille de détergent (solide) avec de l'eau, directement dans le récipient 610.

### FONCTION NETTOYAGE - côté dispositif

Le dispositif 100 est configuré pour passer d'une configuration d'analyse d'urine à une configuration de nettoyage, et vice et versa. Dans la configuration d'analyse d'urine, le dispositif 100 est configuré pour être placé dans la cuvette 106 des toilettes, pour collecter et analyser l'urine d'un utilisateur, comme représenté sur la figure 1 et expliqué en détail ci-dessus.

Le dispositif 100 est configuré pour passer en configuration de nettoyage lorsque le dispositif 100 est placé dans le récipient 610, en configuration opérationnelle de l'ensemble 700. Le dispositif d'analyse d'urine 100 est alors configuré pour lancer une phase de nettoyage dans laquelle le circuit fluidique 230 est en position de prélèvement. Le dispositif 100 est alors configuré pour aspirer le liquide de nettoyage 710 présent dans le récipient 610, via l'orifice de collecte 218 au moyen de la pompe 780 du circuit fluidique 230. Le dispositif 100 est configuré pour faire circuler le liquide de nettoyage 710 dans le circuit fluidique 230 et pour purger le liquide de nettoyage 710 par l'orifice de drainage 530. Une phase de nettoyage peut comprendre une pluralité de cycles successifs d'aspiration et de purge.

En particulier, en configuration de nettoyage, le circuit fluidique 230 comprend en série l'orifice de collecte 218, l'injecteur et l'orifice de drainage 530. Le circuit fluidique 230 communique ainsi entre l'orifice de collecte 218 et l'orifice de drainage 530. Lors de la phase de nettoyage, le liquide de nettoyage 710 parcourt donc le circuit fluidique 230, depuis l'orifice de collecte 218 jusqu'à l'orifice de drainage 530, en passant par l'injecteur, permettant ainsi leur nettoyage.

En effet, lors du fonctionnement du dispositif 100 en configuration d'analyse d'urine, de l'urine circule de manière répétée dans le circuit fluidique 230. Des dépôts peuvent alors se produire pouvant conduire à obstruer en partie le circuit fluidique et/ou à contaminer les urines suivantes et donc fausser les analyses futures. Le nettoyage par la station de nettoyage 600 du circuit fluidique 230 du dispositif 100 permet alors de nettoyer ces dépôts et augmenter ainsi la durée de vie du dispositif 100 ainsi que fiabiliser les analyses.

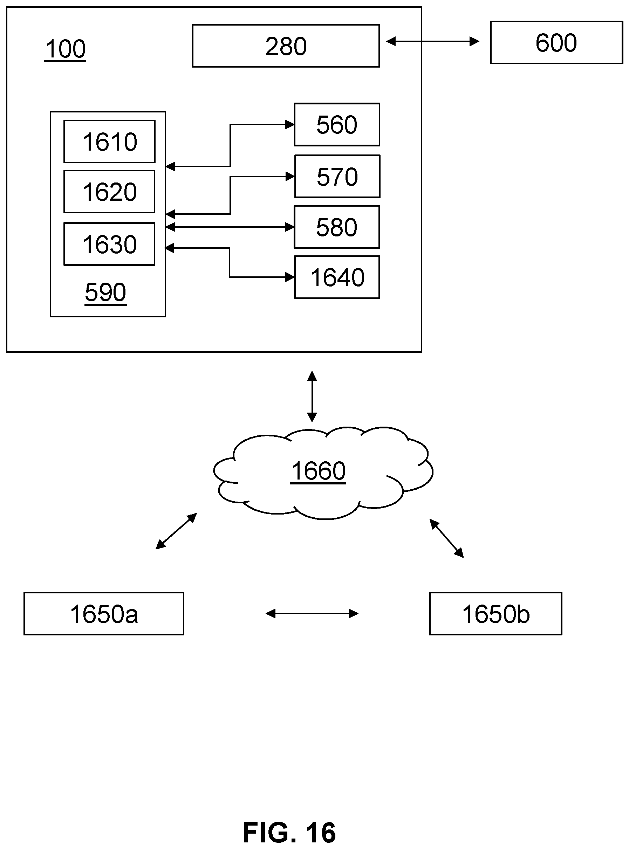

Dans un mode de réalisation, la phase de nettoyage est contrôlée par une circuiterie de contrôle 590 du dispositif 100, représenté plus en détail sur la figure 16. La circuiterie de contrôle 590 comprend un processeur 1610, une mémoire 1620 et une interface I/O 1630 (entrée/sortie, « *input*/*output* ») configurée pour envoyer et recevoir des données depuis la circuiterie de contrôle 590. Un module de communication 1640 peut être prévu pour échanger des données avec un terminal externe 1650. Le module de communication 310 peut être un module sans fil, de type Wi-Fi, Bluetooth, Bluetooth Low Emission, etc. La circuiterie de contrôle 590 peut notamment échanger avec le capteur radar 570, le capteur de température 560 et/ou le capteur de liquide 580 pour contrôler la phase de nettoyage.

Le système d'alimentation électrique 280 est configuré pour alimenter ces composants en énergie.

La mémoire 1620 peut stocker des instructions, qui, lorsqu'exécutées par le processeur 1610, mettent en oeuvre la ou les méthodes de la présente description. Les méthodes sont préférablement effectuées en local, par le processeur 1610 du dispositif 100.

Le dispositif 100 peut communiquer, à l'aide du module de communication 1640 et à l'aide d'un réseau de communication 1660, avec un terminal externe 1650, de type terminal mobile 1650a (« *smartphone* »). Le dispositif 100 peut aussi communiquer avec un serveur 1650b, soit directement via le réseau de communication 1660 soit via le terminal externe mobile 1650a.

En particulier, la circuiterie de contrôle 590 est configurée pour activer la pompe 780 lorsque le dispositif 100 est placé dans le récipient 610 afin d'aspirer le liquide de nettoyage 710 en configuration de nettoyage. La circuiterie de contrôle 590 est configurée pour commander l'alimentation en énergie de la pompe 780 par le dispositif d'alimentation électrique 280 du dispositif 100.

La configuration de nettoyage du dispositif 100 peut être activée au moyen d'un signal d'activation envoyé par l'utilisateur via un terminal externe 1650, par exemple via un smartphone ou un serveur.

Dans un mode de réalisation, la circuiterie de contrôle 590 est configurée pour activer la pompe 780 lorsque la circuiterie de contrôle 590 détermine que des conditions de nettoyage prédéterminées sont remplies. Les conditions de nettoyage comprennent par exemple la réception d'un signal envoyé par le système d'alimentation électrique 280 indiquant que ce dernier reçoit de l'énergie de la part du chargeur 620. En variante ou en complément, les conditions de nettoyage comprennent la réception d'un signal envoyé par le capteur radar 570 indiquant que la paroi 630 du récipient 610 est détectée. En variante ou en complément, les conditions de nettoyage comprennent la réception d'un signal envoyé par le capteur de température 560 indiquant qu'un changement de température sur le boitier 204 a été détecté, témoignant de la présence de liquide de nettoyage 710 dans le récipient 610. En variante ou en complément, les conditions de nettoyage comprennent la réception d'un signal depuis le terminal externe 1650 (par exemple un déclenchement par l'utilisateur d'une session de nettoyage).

Dans un mode de réalisation, la circuiterie de contrôle 590 est configurée pour suspendre le fonctionnement de la pompe 780 lorsque la circuiterie de contrôle 590 reçoit un signal du capteur de liquide indiquant que le fluide aspiré est du gaz ou un fluide mixte, et non du liquide, témoignant d'une absence ou d'un manque de liquide de nettoyage 710 dans le récipient 610.

### FONCTION CHARGE - côté station

Le chargeur 620 est configuré pour alimenter en énergie le dispositif d'analyse d'urine 100 lorsque l'ensemble 700 est dans la configuration opérationnelle. La charge du dispositif 100 peut s'effectuer dès que le dispositif 100 est inséré dans le récipient 610. En variante, la station 600 peut comprendre un bouton de commande actionnable par l'utilisateur pour lancer ou arrêter la charge du dispositif 100.

Le chargeur 620 est logé au niveau du récipient 610. Autrement dit, le chargeur 620 est logé à l'intérieur du volume interne 640 du récipient ou le chargeur 620 est logé entre les deux cloisons formant la paroi 630 du récipient 610.

Le chargeur 620 est placé en vis-à-vis de la paroi 630 du récipient 610. Comme visible sur la figure 8, le chargeur 620 peut être disposé dans la saillie latérale 722 formée dans la paroi 630. Le chargeur 620 est positionné, le long de la profondeur du récipient 610 selon l'axe C, au moins en partie entre le fond 740 du récipient 610 et la jauge 750.

Dans un mode de réalisation, le chargeur 620 est un chargeur sans fil, c'est-à-dire que la charge entre le chargeur 620 et le dispositif 100 est sans-fil. Autrement dit, il n'y a pas de connexion physique électrique connectant le dispositif 100 au chargeur 620. Le chargeur 620 est notamment un chargeur à induction électromagnétique. En particulier, le chargeur 620 comprend une bobine. Les inventeurs ont remarqué que la charge sans fil était possible malgré la présence de liquide de nettoyage dans le récipient et donc entre la paroi 630 et le dispositif 100.

En variante non représentée, le chargeur 620 comprend un port de charge connectable au dispositif d'analyse d'urine 100, par exemple un port USB faisant saillie de la paroi 630 dans le volume interne 640.

La station de nettoyage 600 peut comprendre un câble de connexion électrique 660 pour connecter le chargeur 620 à un réseau électrique. Le câble 660 fait par exemple saillie depuis le bas de l'extérieur du récipient 620. Le câble 660 peut être amovible, par exemple via une connexion USB.

En variante ou en complément, la station de nettoyage 600 comprend une batterie configurée pour alimenter le chargeur 620. La batterie peut être rechargée via le câble de connexion électrique 650. En variante, la batterie se présente sous la forme de piles à remplacer.

### FONCTION CHARGE - côté dispositif

Dans un mode de réalisation, le système d'alimentation électrique 280 est alimenté par le chargeur 620, qu'il y ait du liquide de nettoyage ou non dans le récipient 610. La station de nettoyage 660 fonctionne ainsi comme un simple chargeur.

Dans un mode de réalisation, le système d'alimentation électrique 280 est configuré pour être alimenté en énergie par le chargeur 620 et pour fournir simultanément de l'énergie au dispositif 100. Ainsi, l'activation de la pompe 780 peut être alimentée par de l'énergie fournie *in* fine par le chargeur 620 et se faire sans décharger la batterie (et ainsi sans réduire la durée d'utilisation du dispositif 100 dans les toilettes). De plus, lorsque la consommation de la pompe est inférieure à la puissance du chargeur, le système d'alimentation électrique 280 peut charger la batterie, malgré le nettoyage du circuit fluidique par la pompe 780.

La batterie est disposée dans le dispositif 100 de sorte à être placé en vis-à-vis du chargeur 620 dans la configuration opérationnelle. En particulier, la batterie est disposée en vis-à-vis de la face du boitier 204 en contact avec la saillie latérale 720 logeant le chargeur 620.

Lorsque l'ensemble 700 est en configuration opérationnelle, l'indicateur lumineux 290 du dispositif 100 est configuré pour fournir une information visuelle relative à l'état de charge de la batterie. Cette information peut être observé par l'utilisateur, ce qui lui permet de savoir lorsque la charge de la batterie est complète.

Dans une variante, le système d'alimentation électrique 280 ne comprend pas de moyens de stockage de l'énergie. L'énergie fournie par le chargeur 620 est alors directement transmise au fonctionnement du dispositif 100, notamment à la pompe.

### METHODE

Une méthode de nettoyage du dispositif 100 au moyen de la station de nettoyage 600 va désormais être décrite.

L'ensemble 700 est initialement dans la configuration séparée. Le dispositif d'analyse d'urine 100 est par exemple placé dans la cuvette de toilettes 106 de l'utilisateur, comme représenté sur la figure 1. Le dispositif 100 est alors en configuration d'analyse d'urine et peut réaliser régulièrement des analyses de l'urine de l'utilisateur.

Puis, lorsque par exemple la cartouche 202 est consommée ou que le boitier 204 a un aspect visuel sale ou encore lorsque le niveau de charge de la batterie est bas, l'utilisateur peut décider de procéder à un nettoyage du dispositif 100.

L'utilisateur remplit le récipient 620 avec du liquide de nettoyage 710, typiquement jusqu'à la jauge 750.

Dans un mode de réalisation, l'utilisateur retire alors le dispositif 100 des toilettes, par exemple en saisissant le bras de fixation 500 et insère le dispositif 100 dans le récipient 620, comme représenté sur la figure 10. En variante, l'utilisateur insère le récipient 610 autour du dispositif 100 dans la cuvette 106 de toilettes, comme représenté sur la figure 15.

Le dispositif 100 est alors au moins partiellement immergé dans le liquide de nettoyage 710. Notamment l'orifice de collecte 218 est immergé dans le liquide de nettoyage 710. L'utilisateur peut en outre refermer le récipient 610 avec le couvercle 900. L'ensemble 700 passe alors de la configuration séparée à la configuration opérationnelle. Le couvercle 900 passe de la configuration ouverte à la configuration fermée.

La partie du boitier 204 au contact avec le liquide de nettoyage 710, et dans le cas échéant, le bras de fixation 500 sont alors nettoyés de manière passive.

Le chargeur 620 alimente le dispositif 100 en énergie, notamment le système d'alimentation électrique 280. Dans un mode de réalisation, le chargeur 620 alimente le dispositif 100 de manière sans fil, par induction électromagnétique. Le système d'alimentation électrique 280 peut comprendre une batterie qui est alors rechargée. L'utilisateur peut suivre l'état de la charge de la batterie via l'indicateur lumineux 290 du dispositif 100, visible à travers le couvercle 900.

En parallèle ou successivement, l'utilisateur envoie via un terminal externe 1-650 un signal d'activation au dispositif 100 pour le faire passer de la configuration d'analyse d'urine à la configuration de nettoyage.

Puis, la circuiterie de contrôle 590 du dispositif 100 lance un premier cycle de la phase de nettoyage en activant la pompe avec l'énergie fournie par le chargeur 620 et/ou avec la batterie du dispositif 100 lorsque que les conditions de nettoyage prédéterminées sont remplies. Les conditions de nettoyage comprennent notamment la réception d'un signal envoyé par le système d'alimentation électrique 280 indiquant qu'il reçoit de l'énergie de la part du chargeur 620 et/ou la réception d'un signal envoyé par le capteur radar 570 indiquant que la paroi 630 du récipient 610 est détectée, et/ou la réception d'un signal envoyé par le capteur de température 560 indiquant qu'un changement de température sur le boitier 204 a été détecté, témoignant de la présence de liquide de nettoyage 710 dans le récipient 610.

La pompe aspire alors le liquide de nettoyage 710 par l'orifice de collecte 218. Le liquide de nettoyage 710 circule et nettoie le circuit fluidique 230 jusqu'à être purger par l'orifice de drainage 530.

La circuiterie de contrôle 590 suspend le fonctionnement de la pompe si la circuiterie de contrôle 590 reçoit un signal du capteur de liquide indiquant que le fluide aspiré est du gaz ou un fluide mixte, et non du liquide, témoignant d'une absence ou d'un manque de liquide de nettoyage 710 dans le récipient 610.

La phase de nettoyage peut comprendre plusieurs cycles de pompage et de drainage du liquide de nettoyage 710. La phase de nettoyage peut s'arrêter après une durée prédéterminée, par exemple entre cinq minutes et cinq heures, ou lorsque l'utilisateur retire le dispositif 100 du récipient 610, par exemple lorsque la charge de la batterie est complète.

Le nettoyage et la charge du dispositif d'analyse d'urine 100 est donc effectué de manière simple et intuitive. Il suffit à l'utilisateur d'insérer le dispositif 100 dans le récipient 610 pour que le chargeur 620 puisse fournir de l'énergie au dispositif 100 et que le liquide de nettoyage 710 nettoie l'extérieur au moins une partie du boitier 204. Le dispositif 100 peut en outre aspirer du liquide de nettoyage 710 et le faire circuler dans le circuit fluidique 230 afin de le nettoyer. La station de nettoyage 600 peut donc permettre un nettoyage extérieur, intérieur ainsi qu'une recharge du dispositif 100, et permet donc d'améliorer la durée de vie et la qualité des mesures effectuées par le dispositif 100.

## Revendications

1. Ensemble (700) comprenant :
- un dispositif d'analyse d'urine, le dispositif d'analyse d'urine (100) comprenant un boitier (204) et un circuit fluidique (230) logé à l'intérieur du boîtier (204), et configuré pour faire circuler de l'urine dans le dispositif d'analyse d'urine (100), le circuit fluidique (230) comprenant un orifice de collecte (218) sur le boitier (204),
- une station de nettoyage (600) pour dispositif d'analyse d'urine, la station de nettoyage (600) comprenant :
• un récipient (610) configuré pour recevoir un liquide de nettoyage (710), le récipient (610) étant en outre dimensionné pour recevoir au moins en partie le dispositif d'analyse d'urine (100), de sorte que le dispositif d'analyse d'urine (100) soit au contact du liquide de nettoyage (710),
• un chargeur (620) configuré pour alimenter en énergie le dispositif d'analyse d'urine (100) lorsque le dispositif d'analyse d'urine (100) est reçu au moins en partie dans le récipient (610).

2. Ensemble (700) selon la revendication 1, dans laquelle le dispositif d'analyse d'urine (100) comprend une batterie rechargeable et le chargeur (620) est configuré pour charger électriquement la batterie.

3. Ensemble (700) selon la revendication 1 ou 2, dans laquelle le chargeur (620) est un chargeur sans fil, de sorte que la charge entre le chargeur (620) et le dispositif d'analyse d'urine (100) est sans fil.

4. Ensemble (700) selon l'une quelconque des revendications précédentes, dans laquelle le récipient (610) comprend au moins une saillie (720, 722, 1300) configurée pour supporter le dispositif d'analyse d'urine (100).

5. Ensemble (700) selon la revendication 4, dans laquelle le dispositif (100) est uniquement soutenue par la ou les saillies (720, 722, 1300) dans le récipient (610).

6. Ensemble (700) selon la revendication 4 ou 5, dans laquelle l'au moins une saillie (720, 722, 1300) définit un volume de dégagement (760) prévu pour recevoir l'orifice de collecte (218) du dispositif d'analyse (100).

7. Ensemble (700) selon l'une quelconque des revendications précédentes, dans laquelle la station de nettoyage (600) comprend un couvercle (900) configuré pour fermer au moins en partie le récipient (610).

8. Ensemble (700) selon la revendication 7, dans laquelle le couvercle (900) comprend au moins une cale (910) faisant saillie du couvercle (900) vers le récipient (610), chaque cale (910) étant configurée pour maintenir le dispositif d'analyse d'urine (100) en position dans le récipient (610).

9. Ensemble (700) selon la revendication 7 ou 8, dans laquelle le couvercle (900) comprend au moins une ouverture (920) apte à être traversée par un bras de fixation (500) du dispositif d'analyse d'urine (100).

10. Ensemble (700) selon l'une quelconque des revendications précédentes, dans lequel le récipient (610) est dimensionné pour que l'orifice de collecte (218) soit immergé dans le liquide de nettoyage (710) en configuration opérationnelle.

11. Ensemble (700) selon l'une quelconque des revendications précédentes, dans lequel le récipient (610) est formé par une paroi (630) et un fond (740), le récipient (610) étant dimensionné pour que l'orifice de collecte (218) soit à l'écart d'au moins l'un parmi la paroi (630) et le fond (740).

12. Ensemble (700) selon l'une quelconques des revendications précédentes, dans lequel le circuit fluidique (230) comprend une pompe (780) configurée pour aspirer du liquide dans le circuit fluidique (230) via l'orifice de collecte (218).

13. Ensemble (700) selon la revendication 12, dans lequel le chargeur (620) est configuré pour, en configuration opérationnelle, alimenter la pompe (780) du circuit fluidique (230), de sorte que le liquide de nettoyage (710) soit aspiré dans le circuit fluidique (230).

14. Ensemble (700) selon l'une quelconques des revendications précédentes, dans lequel le chargeur (620) est en outre configuré pour recharger une batterie du dispositif d'analyse d'urine (100).

15. Ensemble (700) selon l'une quelconques des revendications précédentes, dans lequel le dispositif d'analyse d'urine (100) est configuré pour passer d'une configuration d'analyse d'urine à une configuration de nettoyage, le dispositif (100) comprenant au moins un réactif propre à réagir avec de l'urine, le circuit fluidique (230) comprenant un orifice de drainage (530) sur le boitier (204),
le circuit fluidique (230) étant configuré pour, dans la configuration d'analyse d'urine, injecter le fluide collecté par l'orifice de collecte (218) sur le réactif,
le circuit fluidique (230) étant configuré pour, dans la configuration de nettoyage, faire circuler le fluide collecté par l'orifice de collecte (218) directement vers l'orifice de drainage (530).
